(19) Europäisches Patentamt
European Patent Office
Office européen des brevets

(11) Numéro de publication: **0 254 623 B1**

(12) # FASCICULE DE BREVET EUROPEEN

(45) Date de publication de fascicule du brevet: **09.06.93**

(21) Numéro de dépôt: **87401585.2**

(22) Date de dépôt: **07.07.87**

(51) Int. Cl.5: **C07D 471/04**, A61K 31/435, A61K 31/495, A61K 31/535, A61K 31/54, C07D 295/06, //(C07D471/04,249:00,221:00)

(54) **Nouveaux dérivés amino alkyl thio de triazolopyridine ou triazoloquinoline, leurs procédés de préparation, médicaments les contenant, utiles notamment comme antalgiques.**

(30) Priorité: **23.07.86 FR 8610709**

(43) Date de publication de la demande:
**27.01.88 Bulletin 88/04**

(45) Mention de la délivrance du brevet:
**09.06.93 Bulletin 93/23**

(84) Etats contractants désignés:
**AT BE CH DE ES FR GB GR IT LI LU NL SE**

(56) Documents cités:
**US-A- 3 050 525**
**US-A- 4 419 516**

(73) Titulaire: **LABORATOIRES UPSA**
**1 bis, rue du Docteur Camille Bru**
**F-47000 Agen(FR)**

(72) Inventeur: **Bru-Magniez, Nicole, D. médecine**
**24/26, avenue Raphael**
**F-75016 Paris(FR)**
Inventeur: **Teulon, Jean-Marie**
**13, avenue Guibert**
**F-78110 La Celle St Cloud(FR)**
Inventeur: **Launay, Michèle**
**29, avenue du Mont Valérien**
**F-92500 Rueil-Malmaison(FR)**

(74) Mandataire: **Portal, Gérard et al**
**Cabinet Beau de Loménie 158, rue de l'Université**
**F-75340 Paris Cédex 07 (FR)**

EP 0 254 623 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne des dérivés d'amino alkyl thio de triazolopyridine ou triazoloquinoline de formule (I). Elle concerne également les procédés de préparation des dits produits et leurs applications en thérapeutique. Elle concerne en outre les composés intermédiaires nouveaux permettant la synthèse des dits produits.

Les nouveaux composés selon l'invention sont choisis parmi l'ensemble constitué par les composés de formule générale (I) et leurs sels d'addition d'acides non toxiques.

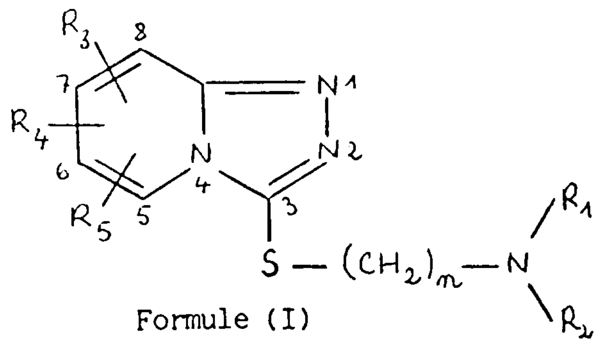

Formule (I)

dans laquelle:

- n représente un nombre entier compris entre 1 et 8 : optimalement 2 ou 3 : $(CH_2)_n$-N-$R_1$ peut également former un hétérocycle à 6 atomes ;
- $R_1$ et $R_2$ peuvent représenter indépendamment l'hydrogène, un alkyle inférieur de 1 à 5 atomes de carbone, ou former ensemble avec l'atome d'azote un cycle choisi parmi la pipéridine, la morpholine, la phényl tétrahydro pyridine, la pipérazine, la pipérazine N-substituée par un phényle ou un hétérocycle azoté choisi parmi pyridine et pyrimidine ; dans le cas des phényl tétrahydropyridines et des pipérazines substituées par un phényl ou un hétérocycle azoté choisi parmi pyridine et pyrimidine, le phényl ou l'hétérocycle azoté peut être substitué par des halogènes , des groupements méthoxy, hydroxy, nitro, amino, cyano, méthyle, trifluorométhyle et trichlorométhyle ;
- $R_3$, $R_4$ et $R_5$ peuvent représenter indépendamment l'hydrogène, un méthyle, un hydroxy benzyle, un halogène, un trifluorométhyle, ou deux d'entre eux peuvent former un phényle dans le cas des triazoloquinolines ;

ainsi que les sels d'addition d'acide non toxiques
ou un composé choisi parmi :

a) Dichlorhydrate hydraté de 3-[2-(4-(2,4-difluorophényl) pipérazin -1-yl) éthyl mercapto]8-(1-hydroxy éthyl)-1,2,4-triazolo-(4,3a)-pyridine ;
b) 3-[2-(4-(diphényl méthyl) pipérazin-1-yl) éthyl mercapto]-1,2,4-triazolo-(4,3a)-pyridine ; et
c) 3-[(1-benzoyl pipéridin-4-yl) mercapto]-1,2,4,-triazolo-(4,3a)-pyridine.

Certains composés particuliers sont ceux pour lesquels $R_3 = R_4 = R_5$ = l'hydrogène.

Les composés préférés selon l'invention sont ceux pour lesquels n = 2 ou 3.

D'autres composés préférés sont ceux pour lesquels $R_3$ = trifluorométhyle et $R_4 = R_5$ = l'hydrogène.

D'autres composés préférés sont ceux pour lesquels $NR_1R_2$ peut représenter :

- la 4-(3-trifluorométhylphényle)-pipérazine ;
- la 4-(2,4-difluorophényle)-pipérazine ;
- la 4-(3-chlorophényle)-pipérazine ;
- la 4-(2-fluorophényle)-pipérazine .

Les composés encore davantage préférés sont ceux qui répondent aux formules suivantes :

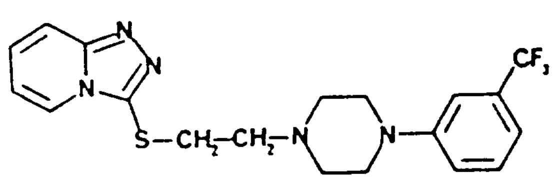

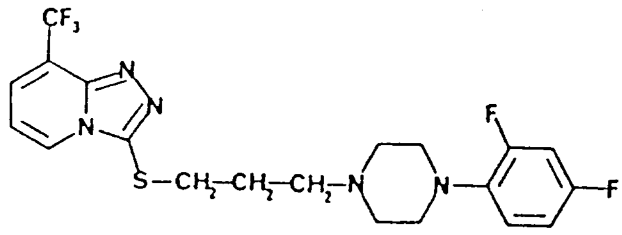

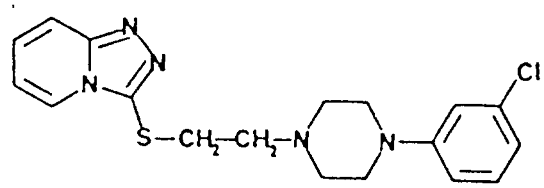

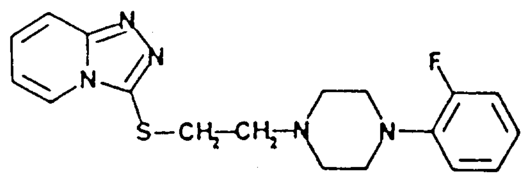

L'invention couvre également à titre de produits nouveaux la 4-(2,4-difluorophényle)-pipérazine ou un de ses sels d'addition, de préférence un de ses sels d'addition d'acide non toxique ou pharmaceutiquement acceptable, ce produit constituant un intermédiaire très utile pour la préparation des produits de formule (I) ci-dessus.

La présente invention couvre également l'utilisation des composés de formule (I) précitée ainsi que la 4-(2,4-difluoro phényle)-pipérazine ou un de ses sels d'additions d'acide non toxique comme médicament doué de propriétés antalgiques et agissant notamment sur le système nerveux central comme anxiolytique.

L'invention couvre également un procédé de préparation des composés de formule (I) par action des dérivés thio de formule (II):

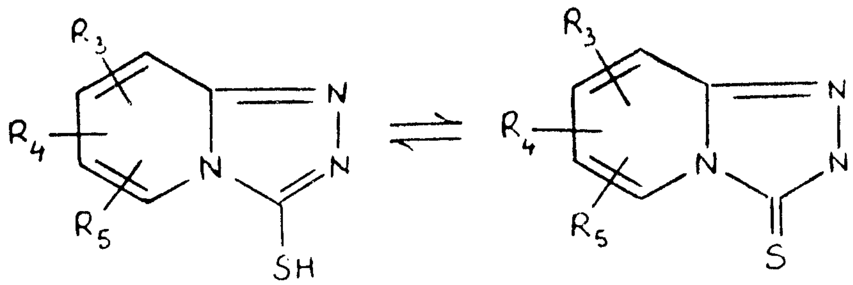

Formule (II)

dans laquelle $R_3$, $R_4$ et $R_5$ sont définis comme ci-dessus sur des halogéno alkylamines ou des tosyloxyalkylamines, ou des mesyloxyalkylamines de formule (III)

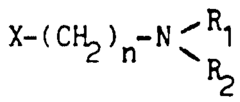

Formule (III)

dans laquelle n, $R_1$ et $R_2$ sont définis comme ci-dessus, X étant un halogène ou un bon groupement partant, tel que tosyloxy ou mesyloxy.

Cette réaction peut se faire en métallant la fonction thiol par un agent classique de métallation tel que l'hydrure de sodium ou de lithium, la soude ou la potasse, un carbonate de sodium ou de potassium, ou en présence d'une base telle que par exemple la triéthylamine ou la pyridine dans un solvant organique usuel tel que le benzène ou le toluène, un alcool tel que le méthanol ou l'éthanol, le diméthylformamide, la N-méthylpyrrolidone ou le diméthylacétamide à une température comprise entre 20 et 150 °C.

Les composés de formule (I) peuvent également être préparés par action d'un composé de formule (IV)

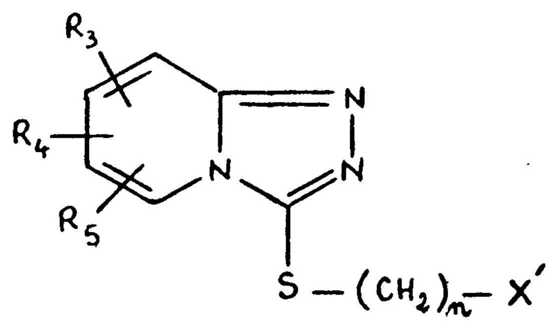

**Formule (IV)**

dans laquelle $R_3$, $R_4$, $R_5$ et n sont définis comme ci-dessus, X' étant un halogène ou un bon groupement partant tel que tosyloxy ou mesyloxy sur une amine de formule (V)

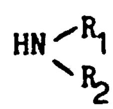

**Formule (V)**

dans laquelle $R_1$ et $R_2$ sont définis comme ci-dessus, dans un solvant organique tel que toluène ou xylène, ou un alcool en présence d'une molécule supplémentaire d'amine ou de la triéthylamine ou de la pyridine entre 50 et 140 °C.

Les composés de formule (IV) peuvent être préparés par action de dihalogéno alkanes sur les composés de formule (II) dans les mêmes conditions que les halogèno alkylamines, ou par chloration ou bromation par des méthodes classiques, telles que le chlorure de thionyle par exemple, des alcools correspondants de formule (VI)

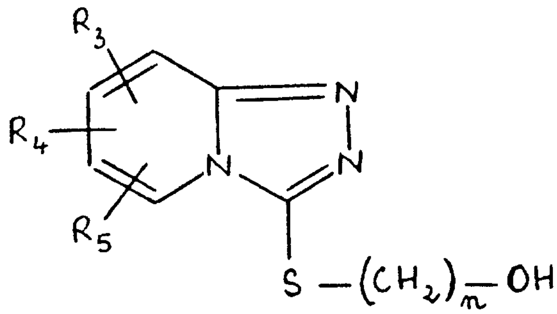

Formule (VI)

dans laquelle $R_3$, $R_4$, $R_5$ et n sont définis comme ci-dessus eux-mêmes préparés par action d'halogéno alcools sur les composés de formule (II) dans les mêmes conditions que les halogéno alkylamines. Les dérivés de formule (IV) dans lesquels X' est un groupement tosyloxy ou mesyloxy, sont obtenus par action du chlorure de tosyle ou du chlorure de mésyle sur les dérivés de formule (VI).

Certains dérivés de formule (I) dans lesquels $NR_1R_2$ forme une phényl ou hétéroaryl pipérazine, peuvent également être obtenus par action d'un halogèno phényle ou d'un halogéno hétéroaryle sur lequel l'halogène est convenablement activé sur le dérivé pipérazinyl NH correspondant dans un solvant organique usuel entre 50 et 150 ° C.

Certains dérivés de formule (II), par exemple celui pour lequel $R_3 = R_4 = R_5 = H$ sont connus dans la littérature.(voir BEILSTEIN, vol 26, complément II -page 86, composés V et VI). Les dérivés non décrits sont préparés selon des méthodes consistant à faire réagir le disulfure de carbone sur les dérivés hydrazino de formule (VII)

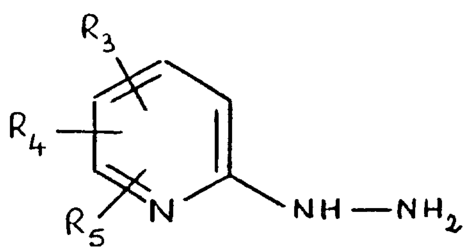

Formule (VII)

dans un solvant tel que la pyridine, à une température comprise entre 20 et 120 ° C, $R_3$, $R_4$ et $R_5$ étant définis comme ci-dessus.Les 2-hydrazino pyridines de formule (VII) sont décrites dans la littérature (voir BEILSTEIN, vol 22, complément I - page 688).

Les dérivés de formule (VII) non décrits sont synthétisés par action de l'hydrazine ou de l'hydrate d'hydrazine sur les dérivés de formule (VIII) dans un solvant tel que les alcools ou sans solvant à une température comprise entre 70 et 150 ° C.

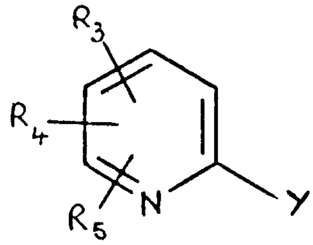

Formule (VIII)

dans la formule (VIII) $R_3$, $R_4$ et $R_5$ sont définis comme ci-dessus, Y représentant un atome d'halogène, en particulier le chlore ou le brome.

Les composés de formule (III) sont préparés par action du chlorure de thionyle ou du chlorure de tosyle ou du chlorure de mesyle, par des méthodes connues en soi sur des alcools de formule (IX)

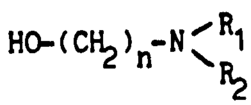

Formule (IX)

dans laquelle $R_1$, $R_2$ et n sont définis comme ci-dessus.

Les alcools de formule (IX) peuvent être préparés, soit par action de l'éthylène oxyde dans le cas où n = 2, soit par action d'un halogèno alcool sur les amines correspondantes de formule (V) selon des méthodes connues en soi.

Certaines amines de formule (V) sont originales et permettent d'obtenir des dérivés de formule (I) particulièrement actifs, ayant elles-mêmes des propriétés antalgiques.

La demanderesse revendique en particulier le dérivé de la pipérazine de formule (X) ou un de ses sels d'addition d'acides non toxiques.

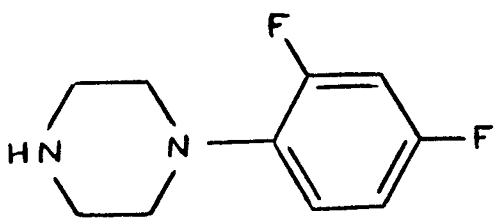

Formule (X)

La difluoro-2,4 phényl pipérazine (X) est obtenue par action de bis halogéno éthyl amine sur la difluoro-2,4 aniline dans un solvant aromatique du type toluène ou xylène, ou un alcool, à une température comprise entre 80 et 150 ° C.

Les sels d'addition des composés de formule (I) ou de formule (X) peuvent s'obtenir par réaction de ces composés avec un acide minéral ou organique suivant une méthode connue en soi. Parmi les acides utilisables à cet effet, on citera les acides chlorhydrique, bromhydrique, sulfurique, phosphorique, 4-toluène sulfonique, méthane sulfonique, cyclohexylsulfa- mique, oxalique, succinique, formique, fumarique, malëique, citrique, aspartique, cinnamique, lactique, glutamique, N-acétylaspartique, N-acétyl glutamique, ascorbique, malique, benzoïque, nicotinique et acétique.

Selon l'invention, on propose des compositions thérapeutiques utiles notamment pour le traitement de la douleur. En outre, ces dérivés possèdent des propriétés intéressantes sur le système nerveux central et en particulier anxiolytiques non sédatives pouvant être bénéfiques dans le traitement de la composante psychique de la douleur. On les revendique donc également pour ces propriétés. Les compositions thérapeutiques proposées sont caractérisées en ce qu'elles renferment en association avec un excipient physiologiquement acceptable au moins un composé de formule (I) ou (X) ou un de ses sels d'addition d'acides non toxiques.

D'autres caractéristiques et avantages de l'invention seront mieux compris à la lecture qui va suivre de quelques exemples de préparation nullement limitatifs mais donnés à titre d'illustration.

Le tableau III pages 52-58 donne la formule développée de certains produits.

Exemple 1 : **4-(2,4-difluoro phényl) pipérazine dichlorhydrate**

**Formule X**

On porte à ébullition durant 14 heures un mélange de 0.1 mole de bromhydrate de bis bromoéthyl amine avec 0.1 mole de 2,4-difluoro aniline dans 50 ml de butanol-1. Le mélange réactionnel est ensuite refroidi et le précipité obtenu est essoré. Les cristaux sont repris à l'eau et on basifie à froid par une solution de soude 10%. La phase aqueuse est extraite au chloroforme qu'on lave a l'eau, sèche sur carbonate de sodium et qu'on filtre. Le chloroforme évaporé, le résidu obtenu est repris a l'acétone. Dans cette solution, on fait barboter du gaz chlorhydrique jusqu'à pH acide sous agitation et on laisse les cristaux formés s'organiser. Les cristaux obtenus sont essorés puis lavés a l'acétone et séchés. On récupère ainsi le dichlorhydrate de 4-(2,4-difluoro phényl) pipérazine sous forme de cristaux de point de fusion 205 ° C.

En calculant la quantité de gaz chlorhydrique ajoutée, on peut également préparer le monochlorhydrate de 4-(2,4-difluoro phényl) pipérazine qui fond à 182 ° C.

Exemple 2 : **2-[4-(2,4-difluorophényl) pipérazin-1-yl] éthanol**

Formule IX : n = 2, 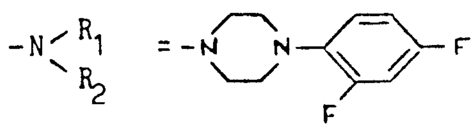

On porte au reflux durant 6 heures une solution de 72 g de 4-(2,4-difluoro phényl) pipérazine et de 50 g de 2-bromo éthanol dans 300 ml de xylène contenant 55 ml de triéthylamine. La solution est ensuite refroidie et additionnée d'éther.
Le précipité de bromhydrate de triéthylamine formé est essoré et le filtrat est concentré sous vide. Le résidu obtenu est alors cristallisé dans un mélange éther isopropylique-pentane. Les cristaux essorés sont lavés avec ce même mélange puis séchés. On récupère ainsi 60 g de 2-[4-(2,4-difluoro phényl) pipérazin-1-yl] éthanol sous forme de cristaux de point de fusion 54 ° C.

Selon le mode opératoire de l'exemple 2, les composés suivants ont été obtenus :

Exemple 3 : **2-[4-(2-méthyl phényl) 1,2,3,6-tétrahydropyridin 1-yl] éthanol**

Formule IX : n = 2, 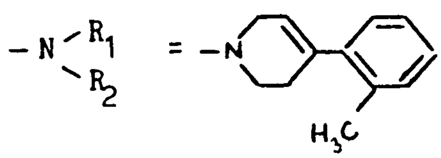

Sous forme d'huile.

Exemple 4 : **2-[4-(3-fluorophényl) pipérazin-1-yl] éthanol**

Formule IX : n = 2,

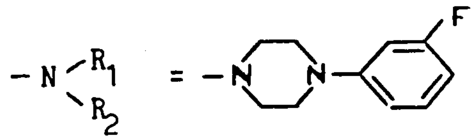

Sous forme de cristaux F = 98 ° C.

Exemple 5 : **2-[4-(2,5-difluoro phényl) pipérazin-1-yl] éthanol**

Formule IX : n = 2,

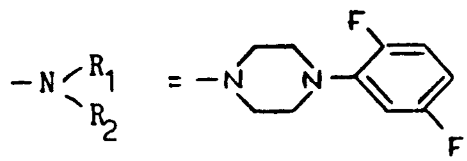

Sous forme d'huile.

Exemple 6 : **3-[4-(2,4-difluoro phényl) pipérazin-1-yl] propanol**

Formule IX : n = 3,

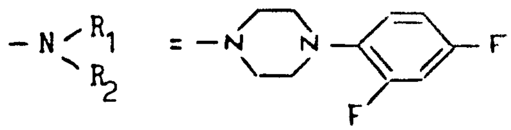

Préparé selon le mode opératoire de l'exemple 2 en utilisant le 3-bromo propanol. 9.4 g de 4-(2,4-difluoro phényl) pipérazine ont conduit à 7.7 g de 3-[4-(2,4-difluoro phényl) pipérazin-1-yl] propanol sous forme de cristaux de point de fusion 88 ° C.

Exemple 7 : **Chlorhydrate de 1-chloro 2-[4-(2,4-difluorophényl) pipérazin- 1-yl] éthane**

Formule III : n = 2,
X = Cl

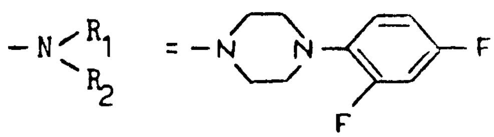

A une solution de 60 g de 2-[4-(2,4-difluoro phényl) pipérazin-1-yl] éthanol préparé à l'exemple 2 dans 300 ml de chloroforme on ajoute goutte à goutte 40 ml de chlorure de thionyle. Après la fin de l'addition, le mélange réactionnel est porté 5 heures au reflux. Après retour à température ambiante, les cristaux formés sont essorés et soigneusement lavés à l'acétone puis séchés. On obtient ainsi 70 g de chlorhydrate de 1-chloro 2-[4-(2,4-difluoro phényl) pipérazin-1-yl] éthane sous forme de cristaux de point de fusion 218 ° C.

Selon le mode opératoire de l'exemple 7, les composés suivants ont été obtenus :

Exemple 8 : **1-chloro 2-[4-(2-méthyl phényl)1,2,3,6-tétrahydro pyridin-1-yl] éthane**

**Formule III :** n = 2, $-N\genfrac{}{}{0pt}{}{R_1}{R_2}$ = 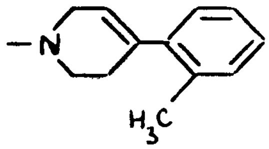
X = Cl

Base libérée à partir de son chlorhydrate : sous forme d'huile.

Exemple 9 : **Dichlorhydrate de 1-chloro 2-[4-(3-fluorophényl) pipérazin-1-yl éthane**

**Formule III :** n = 2, $-N\genfrac{}{}{0pt}{}{R_1}{R_2}$ = 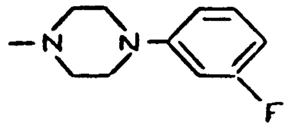
X = Cl

Sous forme de cristaux F = 170-2 °C.

Exemple 10 : **Dichlorhydrate de 1-chloro 2-[4-(2,5-difluoro phényl) pipérazin -1-yl] éthane**

**Formule III :** n = 2, $-N\genfrac{}{}{0pt}{}{R_1}{R_2}$ = 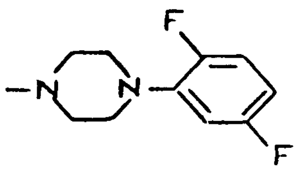
X = Cl

sous forme de cristaux hydratés F = 119-21 °C

Exemple 11 : **Chlorhydrate de 1-chloro 3-[4-(2,4-difluorophényl) pipérazin- 1-yl] propane**

**Formule III :** n = 3, $-N\genfrac{}{}{0pt}{}{R_1}{R_2}$ = 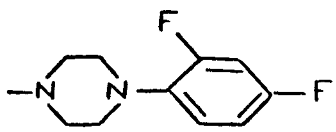
X = Cl

Selon le mode opératoire de l'exemple 7, mais à partir de 7.7 g de 3-[4-(2,4-difluoro phényl) pipérazin-1-yl) propanol préparé à l'exemple 6, on obtient 8.7 g de chlorhydrate de 1-chloro 3-[4-(2,4-difluoro phényl) pipérazin-1-yl] propane sous forme de cristaux de point de fusion 174 °C.

Exemple 12 : **3-trifluoro méthyl 2-hydrazino pyridine**

**Formule VII** : $R_3$ = 3-$CF_3$, $R_4$ = $R_5$ = H

On porte à reflux durant 6 heures une solution de 200 g de 2-chloro 3-trifluorométhyl pyridine dans 500 ml d'éthanol et 300 ml d'hydrate d'hydrazine. La solution est ensuite concentrée sous vide, puis après refroidissement le résidu cristallin est repris à l'eau, essoré, lavé à l'eau et séché. On obtient 145 g de 3-trifluorométhyl 2-hydrazino pyridine sous forme de cristaux de point de fusion 72 °C.

Exemple 13 : **3-méthyl 2-hydrazino quinoline**

**Formule VII** : $R_3$ = 3-$CH_3$, $R_4$-$R_5$ = 5,6-CH = CH-CH = CH

On porte à reflux durant 3 heures 73.5 g de 3-méthyl 2-chloro quinoline dans 102 ml d'hydrate d'hydrazine. Le mélange réactionnel est refroidi, le solide est essoré, lavé à l'eau et séché. La masse cristalline obtenue est recristallisée dans l'isopropanol. On obtient 50.5 g de 2-hydrazino 3-méthyl quinoline de point de fusion 128 °C.

D'une manière identique les composés suivants ont été obtenus :

Exemple 14 : **5-chloro 2-hydrazino pyridine**

**Formule VII :** $R_3$ = 5-Cl, $R_4$ = $R_5$ = H

Sous forme d'huile.

Exemple 15 **: 3-(α-hydroxy benzyl) 2-hydrazino pyridine**

**Formule VII** : $R_3$ = -CHOH-Φ, $R_4$ = $R_5$ = H

Sous forme de cristaux de point de fusion 132 °C.

Exemple 16 : **3-(1-hydroxy éthyl) 2-hydrazino pyridine**

**Formule VII :** $R_3$ = CHOH-$CH_3$, $R_4$ = $R_5$ = H

Sous forme d'huile.

Exemple 17 : **3,5,7-triméthyl 2-hydrazino quinoline**

**Formule VII :** $R_3$ = 3-$CH_3$, $R_4$-$R_5$ = 5,6-$\underset{CH_3}{C}$=CH-$\underset{CH_3}{C}$=CH .

Sous forme de cristaux de point de fusion 117-9 °C.

Exemple 18: **3-mercapto 8-trifluorométhyl (1,2,4)-triazolo (4,3-a) pyridine**

**Formule II** : $R_3$ = 8-$CF_3$, $R_4$ = $R_5$ = H

A 40 g de 3-trifluorométhyl 2-hydrazino pyridine préparé à l'exemple 12 en suspension dans 75 ml de pyridine, on ajoute goutte à goutte sous agitation à température ambiante 16 ml de disulfure de carbone. Après la fin de l'addition, l'agitation est poursuivie 15 minutes à température ambiante puis le mélange réactionnel est chauffé 5 heures à reflux. Après refroidissement, le milieu est concentré sous vide, puis additionné d'eau. Le résidu solide obtenu est essoré, lavé à l'eau, séché.On obtient 40 g de 3-mercapto 8-trifluorométhyl (1,2,4)-triazolo (4,3-a) pyridine sous forme de cristaux de point de fusion 236 °C.

D'une manière identique, les composés suivants ont été obtenus :

Exemple 19 : **3-mercapto 6-chloro (1,2,4)-triazolo (4,3-a) pyridine**

**Formule II :** $R_3$ = 6-Cl, $R_4$ = $R_5$ = H

Sous forme de cristaux de point de fusion supérieur à 260 °C.

Exemple 20 : **3-mercapto 8-(α-hydroxy benzyl) (1,2,4)-triazolo (4,3-a) pyridine**

**Formule II :** $R_3$ = 8-(CHOH-Φ), $R_4$ = $R_5$ = H

Sous forme de cristaux de point de fusion 220 °C.

Exemple 21 : **3-mercapto 8-(1-hydroxy éthyl) (1,2,4)-triazolo (4,3-a) pyridine**

**Formule II** : $R_3$ = 8-(CHOH-$CH_3$), $R_4$ = $R_5$ = H

Sous forme d'huile.

Exemple 22 : **1-mercapto 4-méthyl-s-triazolo (4,3-a)-quinoline**

**Formule II** : $R_3$ = 8-$CH_3$, $R_4$-$R_5$ = 5,6 -CH=CH-CH=CH-

A 50.5 g de 3-méthyl 2-hydrazino quinoline, préparés à l'exemple 13 en solution dans 365 ml de pyridine, on ajoute goutte à goutte à température ambiante 17.5 ml de disulfure de carbone. On observe la formation d'un précipité qui se redissout peu à peu. Après la fin de l'addition la solution est chauffée 1 h 15 à 75 ° C, puis refroidie et versée dans un grand volume d'eau. Le précipité est essoré, lavé à l'eau. Celui-ci est repris à chaud dans une solution diluée de soude qui est filtrée. Le filtrat est extrait au chloroforme puis acidifié avec de l'acide chlorhydrique concentré. Le précipité formé est essoré, lavé à l'eau et séché. On obtient 52 g de 1-mercapto 4-méthyl s-triazolo (4,3-a)-quinoline de point de fusion 268 ° C.

Par une méthode identique, le dérivé suivant a été préparé :

Exemple 23 : **1-mercapto 4,6,8-triméthyl-s-triazolo (4,3-a)-quinoline**

**Formule II :** $R_3$ = 8-$CH_3$, $R_4$-$R_5$ = 6,5 $-\underset{CH_3}{C}H=CH-\underset{CH_3}{C}=CH-$

Sous forme de cristaux de point de fusion supérieur à 260 ° C.

Exemple 24 : **Maléate de 3-[2-[4-(3-trifluorométhyl phényl) pipérazin-1-yl) éthyl mercapto] 8-trifluorométhyl 1,2,4-triazolo (4,3a)-pyridine**

**Formule I :** $R_3$ = 8-$CF_3$, $R_4$ = $R_5$ = H, n = 2

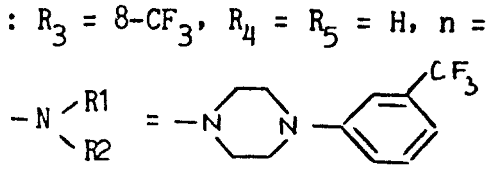

On porte au reflux durant 6 heures une solution de 11 g de 3-mercapto 8-trifluorométhyl-1,2,4-triazolo-(4,3a) pyridine préparés à l'exemple 18 et de 15 g de 1-chloro 2-[4-(3-trifluorométhyl phényl) pipérazin-1-yl] éthane dans 75 ml d'éthanol contenant 8 ml de triéthylamine. Le mélange réactionnel est ensuite concentré sous vide, additionné d'eau et de glace et extrait à l'éther. La phase organique est lavée à l'eau, avec une solution de soude diluée puis encore à l'eau. La phase éthérée est séchée puis concentrée et le résidu obtenu (21.5 g) est dissous dans l'acétone. A cette solution acétonique, on additionne 5.1 g d'acide maléïque dissous dans l'acétone. Les cristaux formés alors sont essorés, lavés à l'acétone et à l'éther et séchés. On obtient ainsi 11.5 g de maléate de 3-[2-(4-(3-trifluorométhyl phényl) pipérazin-1-yl) éthylmercapto] 8-trifluoromethyl 1,2,4-triazolo (4,3a)-pyridine sous forme de cristaux de point de fusion 144-5 ° C.

Selon ce mode opératoire à partir des composés chlorés et des triazolopyridines correspondants, les dérivés suivants ont été obtenus :

Exemple 25 : **Maléate de 3-[2-(4-phenyl 1,2,3,6-tétrahydro pyridin-1-yl) éthyl mercapto] 8-trifluorométhyl 1,2,4-triazolo (4,3a)-pyridine**

Formule I : $R_3 = 8\text{-}CF_3$, $R_4 = R_5 = H$, $n = 2$

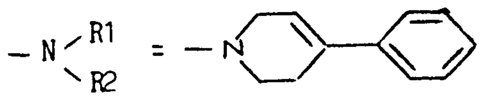

Sous forme de cristaux de point de fusion 174 ° C.

Exemple 26 : **Chlorhydrate de 3-[2-(4-(3-trifluorométhyl phényl) pipérazin- 1-yl) éthyl mercapto] 6-chloro 1,2,4-triazolo-(4,3a)-pyridine**

Formule I : $R_3 = 6\text{-}Cl$, $R_4 = R_5 = H$, $n = 2$

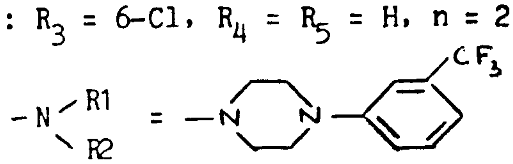

Sous forme de cristaux de point de fusion 227 ° C.

Exemple 27 : **Dichlorhydrate de3-[2-(4-(3-trifluorométhylphényl) pipérazin- 1-yl) éthyl mercapto] 5-methyl 1,2,4-triazolo-(4,3a)-pyridine**

Formule I : $R_3 = 5\text{-}CH_3$, $R_4 = R_5 = H$, $n = 2$

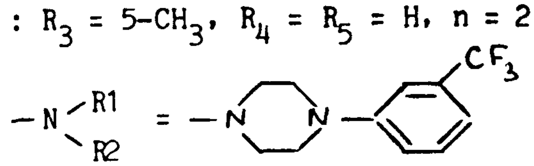

Sous forme de cristaux de point de fusion 230 ° C.

Exemple 28 : **3-[2-(4-(2,4-difluoro phényl) pipérazin-1-yl) éthyl mercapto] 8-trifluorométhyl 1,2,4-triazolo (4,3a)-pyridine**

Formule I : $R_3 = 8\text{-}CF_3$, $R_4 = R_5 = H$, $n = 2$

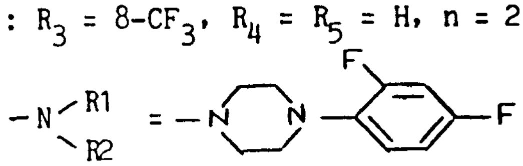

On porte au reflux durant 6 heures une solution de 13 g de 3-mercapto 8-trifluorométhyl-1,2,4-triazolo-(4,3a) pyridine préparés à l'exemple 18 et de 15.5 g de 1-chloro 2-[4-(2,4-difluoro phényl) pipérazin-1-yl] éthane base,libérée de son chlorhydrate préparé à l'exemple 7 par addition de soude et extraction à l'éther, dans 75 ml d'éthanol contenant 10 ml de triéthylamine. Le mélange réactionnel est ensuite concentré sous vide, le résidu est repris au dichlorométhane qu'on lave soigneusement à l'eau, sèche puis qu'on évapore. Le résidu repris dans un mélange d'éther éthylique et d'éther isopropylique cristallise. Les cristaux obtenus sont essorés, lavés à l'éther isopropylique puis séchés. On obtient 15.5 g de 3-[2-(4-(2,4-difluorophenyl) piperazin-1-yl) ethyl mercapto] 8-trifluorométhyl 1,2,4-triazolo-(4,3a)-pyridine sous forme de cristaux de point de fusion 119-120 ° C.

D'une manière identique, à partir des composés de départ correspondants, les dérivés suivants ont été obtenus :

Exemple 29 : **3-[2-(4-(4-fluorophényl) 1,2,3,6-tétrahydropyridin 1-yl) éthyl mercapto] 8-trifluorométhyl 1,2,4-triazolo-(4,3a)-pyridine**

**Formule I** : $R_3 = 8\text{-}CF_3$, $R_4 = R_5 = H$, $n = 2$

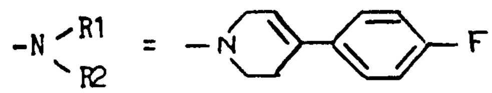

Sous forme de cristaux de point de fusion 138-9 ° C.

Exemple 30 : **3-[2-(4-(4-chlorophényl) 1,2,3,6-tétrahydro pyridin-1-yl)éthyl mercapto] 8-trifluorométhyl 1,2,4-triazolo-(4,3a)-pyridine**

**Formule I** : $R_3 = 8\text{-}CF_3$, $R_4 = R_5 = H$, $n = 2$

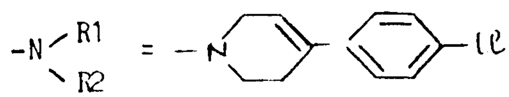

Sous forme de cristaux de point de fusion 126-7 ° C.

Exemple 31 : **Dichlorhydrate de 3-[2-(4-(3-trifluorométhyl phényl) pipérazin -1-yl) éthyl mercapto] 6-méthyl 1,2,4-triazolo-(4,3a)-pyridine**

**Formule I** : $R_3 = 6\text{-}CH_3$, $R_4 = R_5 = H$, $n = 2$

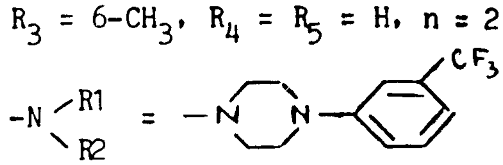

Sous forme de cristaux de point de fusion 205-6 ° C.

Exemple 32 : **3-[3-(4-(2,4-difluoro phényl) pipérazin-1-yl) propyl mercapto] 8-trifluorométhyl 1,2,4-triazolo (4,3a)-pyridine**

**Formule I** : $R_3 = 8\text{-}CF_3$, $R_4 = R_5 = H$, $n = 3$

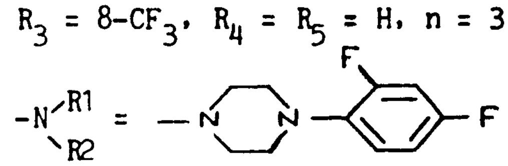

Selon le mode opératoire de l'exemple 28, mais en utilisant 14 g de base de 1-chloro 3-[4-(2,4-difluoro phényl) pipérazin 1-yl] propane préparé à l'exemple 11, et 11.8 g de 3-mercapto 8-trifluorométhyl-1,2,4-triazolo-(4,3a) pyridine préparé à l'exemple 18 on obtient après recristallisation dans l'éther isopropylique 8 g de 3-[3-(4-(2,4-difluoro phényl) pipérazin 1-yl) propyl mercapto] 8-trifluorométhyl-1,2,4-triazolo-(4,3a)-pyridine sous forme de cristaux de point de fusion 88-90 ° C.

Exemple 33 : **Maléate de 3-[2-(4-(3-trifluorométhyl phényl) pipérazin-1-yl) éthyl mercapto] 1,2,4-triazolo (4,3a)-pyridine**

**Formule I :** $R_3 = R_4 = R_5 = H$, $n = 2$

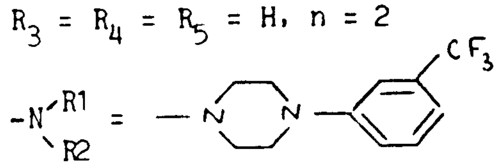

Selon le mode opératoire de l'exemple 24, mais en utilisant 6.6 g de 3-mercapto-1,2,4-triazolo-(4,3a)-pyridine, on obtient après recristallisation dans l'éthanol 15 g du maléate de 3-[2-(4-(3-trifluorométhyl phényl) pipérazin-1-yl) éthyl mercapto]-1,2,4-triazolo-(4,3a)-pyridine sous forme de cristaux de point de fusion 136-138 ° C.

La base cristallise de l'éther et a un point de fusion de 82 ° C.

D'une façon analogue, à partir des dérivés chlorés correspondants, les dérivés suivants ont été obtenus :

Exemple 34 : **Dichlorhydrate de 3-[2-(4-phényl 1,2,3,6-tétrahydro pyridin-1-yl) éthylmercapto] 1,2,4-triazolo (4,3a)-pyridine**

**Formule I :** $R_3 = R_4 = R_5 = H$, $n = 2$

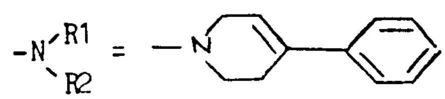

Sous forme de cristaux de point de fusion 250-4 ° C.

Exemple 35 : **Dichlorhydrate de 3-[2-(1,2,3,4-tétrahydro isoquinolin-2-yl) éthyl mercapto] 1,2,4-triazolo (4,3a)-pyridine**

**Formule I :** $R_3 = R_4 = R_5 = H$, $n = 2$

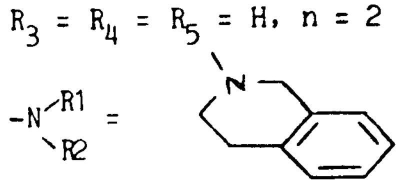

Sous forme de cristaux de point de fusion 218-220 ° C.

Exemple 36 : **Trichlorhydrate, hydrate de 3-[2-(4-(2-méthoxy phényl) pipérazin-1-yl) éthyl mercapto] 1,2,4-triazolo-(4,3a)-pyridine**

**Formule I :** $R_3 = R_4 = R_5 = H$, $n = 2$

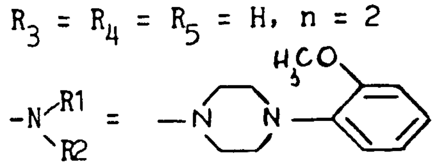

Sous forme de cristaux de point de fusion 218 ° C.

Exemple 37 : **Dichlorhydrate de 3-[2-(4-(2-méthyl phényl) pipérazin-1-yl) éthyl mercapto] 1,2,4-triazolo (4,3a)- pyridine**

Formule I : $R_3 = R_4 = R_5 = H$, $n = 2$

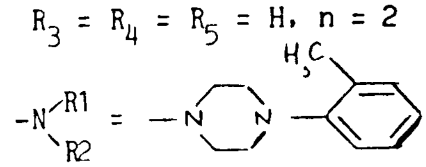

Sous forme de cristaux de point de fusion 228 ° C.

Exemple 38 : **Dichlorhydrate hydraté de 3-[2-(4-(2,4-difluoro phényl) pipérazin-1-yl) éthyl mercapto] 8-(1-hydroxy éthyl)-1,2,4-triazolo-(4,3a)-pyridine**

Formule I : $R_3 = CHOH\text{-}CH_3$, $R_4 = R_5 = H$, $n = 2$

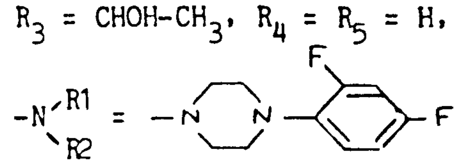

Sous forme de cristaux décomposant à partir de 120 ° C.

Exemple 39: **Trichlorhydrate de 3-[2-(4-(4-fluoro phényl) pipérazin-1-yl) éthyl mercapto]-1,2,4-triazolo (4,3a)-pyridine**

Formule I : $R_3 = R_4 = R_5 = H$, $n = 2$

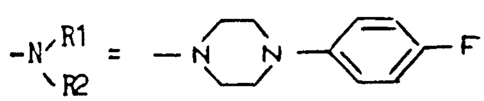

Sous forme de cristaux de point de fusion 219-220 ° C.

Exemple 40 : **Dichlorhydrate de 3-[2-(4-(2-chloro phényl) pipérazin-1-yl) éthyl mercapto]-1,2,4-triazolo (4,3a)-pyridine**

Formule I : $R_3 = R_4 = R_5 = H$, $n = 2$

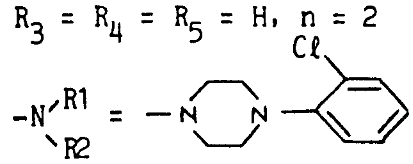

Sous forme de cristaux de point de fusion 181-2 ° C.

Exemple 41 : **Dichlorhydrate de 3-[2-(4-(2-fluoro phényl) pipérazin-1-yl) éthyl mercapto]-1,2,4-triazolo (4,3a)-pyridine**

Formule I : $R_3 = R_4 = R_5 = H$, $n = 2$

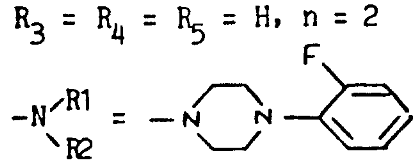

Sous forme de cristaux de point de fusion 215 ° C.

Exemple 42 : **Trichlorhydrate de 3-[2-(4-(4-méthyl phényl) pipérazin-1-yl) éthyl mercapto]-1,2,4-triazolo (4,3a)-pyridine**

Formule I : $R_3 = R_4 = R_5 = H$, $n = 2$

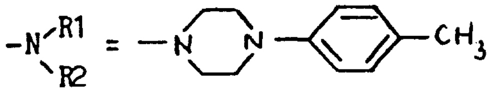

Sous forme de cristaux de point de fusion 203 ° C.

Exemple 43 : **3-[3-(4-(2,4-difluoro phényl) pipérazin-1-yl) propyl mercapto]- 1,2,4-triazolo-(4,3a)-pyridine**

Formule I : $R_3 = R_4 = R_5 = H$, $n = 3$

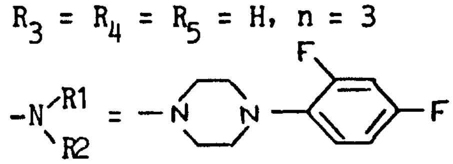

Selon le mode opératoire de l'exemple 28, mais en utilisant 7.7 g de 3-mercapto-1,2,4-triazolo-(4,3a)-pyridine et 14 g de 1-chloro 3-[4-(2,4- difluoro phényl) pipérazin-1-yl] propane, on obtient après recristallisation dans l'éther isopropylique 7.5 g de 3-[3-(4-(2,4-difluoro phényl) pipérazin-1-yl) propylmercapto] 1,2,4-triazolo-(4,3a)-pyridine sous forme de cristaux de point de fusion 77-78 ° C.

Exemple 44 : **3-[2-(4-(2,4-difluoro phényl) pipérazin-1-yl) éthyl mercapto]- 1,2,4-triazolo-(4,3a)-pyridine**

Formule I : $R_3 = R_4 = R_5 = H$, $n = 2$

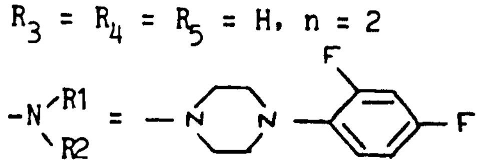

Selon le mode opératoire de l'exemple 28, mais à partir de 9.3 g de 3-mercapto-1,2,4-triazolo-(4,3a)-pyridine on prépare 15 g de 3-[2-(4-(2,4- difluoro phényl) pipérazin-1-yl] éthyl mercapto]-1,2,4-triazolo-(4,3a)- pyridine sous forme de cristaux de point de fusion 98-99 ° C.

D'une façon analogue mais à partir des dérivés chlorés correspondants, on obtient les composés suivants :

Exemple 45 : **3-[2-(4-(4-fluoro phényl) 1,2,3,6-tétrahydro pyridin-1-yl) éthyl mercapto]-1,2,4-triazolo (4,3a)-pyridine**

Formule I : $R_3 = R_4 = R_5 = H$, $n = 2$

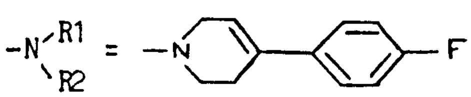

Sous forme de cristaux de point de fusion 87-9 ° C.

Exemple 46 : **Hydrate de 3-[2-(4-(2,4-difluoro phényl) pipérazin-1-yl) éthyl mercapto] 8-(α-hydroxy benzyl)-1,2,4-triazolo-(4,3a)-pyridine**

Formule I : $R_3$ = CHOH-Φ, $R_4$ = $R_5$ = H, n = 2

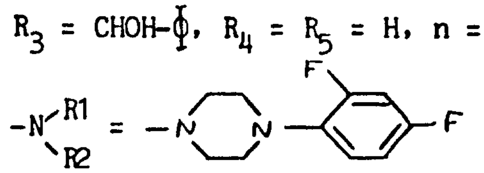

Sous forme de cristaux de point de fusion 88 ° C.

Exemple 47 : **3-[2-(4-(2-méthyl phényl) 1,2,3,6-tétrahydro pyridin-1-yl) éthyl mercapto]-1,2,4-triazolo (4,3a)-pyridine**

Formule I : $R_3$ = $R_4$ = $R_5$ = H, n = 2

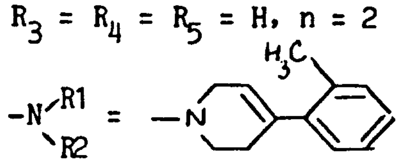

Sous forme de cristaux de point de fusion 54 ° C.

Exemple 48 : **3-[2-(4-(4-chloro phényl) 1,2,3,6-tétrahydro pyridin-1-yl) éthyl mercapto]-1,2,4-triazolo (4,3a)-pyridine**

Formule I : $R_3$ = $R_4$ = $R_5$ = H, n = 2

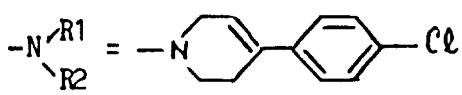

Sous forme de cristaux de point de fusion 126-7 ° C.

Exemple 49 : **3-[2-(4-(3-chloro phényl) pipérazin-1-yl) éthyl mercapto]- 1,2,4-triazolo-(4,3a)-pyridine**

Formule I : $R_3$ = $R_4$ = $R_5$ = H, n = 2

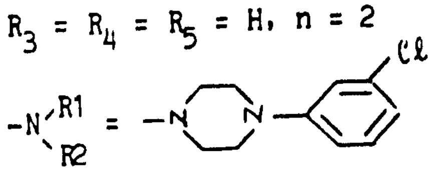

Sous forme de cristaux de point de fusion 115-6 ° C.
Maléate (éthanol) F = 132-3 ° C.
Dichlorhydrate (éthanol) F = 198-9 ° C.

Exemple 50 : **3-[2-(4-(4-méthoxy phényl) pipérazin-1-yl) éthyl mercapto]- 1,2,4-triazolo-(4,3a)-pyridine**

Formule I : $R_3 = R_4 = R_5 = H$, $n = 2$

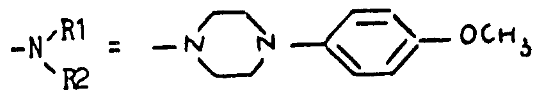

Sous forme de cristaux de point de fusion 111 ° C.

Exemple 51 : **3-[2-(4-(diphényl méthyl) pipérazin-1-yl) éthyl mercapto]- 1,2,4-triazolo-(4,3a)-pyridine**

Formule I : $R_3 = R_4 = R_5 = H$, $n = 2$

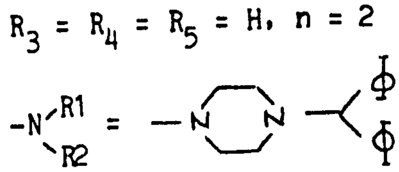

Sous forme de cristaux de point de fusion 103 ° C.

Exemple 52 : **3-[2-(4-(3-fluoro phényl) pipérazin-1-yl) éthyl mercapto]- 1,2,4-triazolo-(4,3a)-pyridine**

Formule I : $R_3 = R_4 = R_5 = H$, $n = 2$

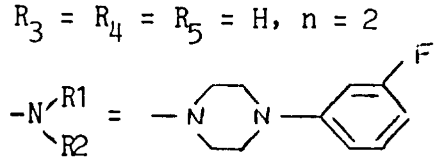

Sous forme de cristaux de point de fusion 105-6 ° C.

Exemple 53 : **3-[2-(4-(1,4-dioxa-8-azaspiro-(4,5)-decan-8-yl) éthylmercapto]- 1,2,4-triazolo-(4,3a)-pyridine**

Formule I : $R_3 = R_4 = R_5 = H$, $n = 2$

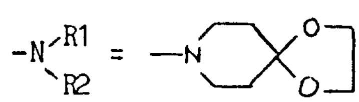

Sous forme de cristaux de point de fusion 128 ° C.

Exemple 54 : **3-[2-(4-phényl pipérazin-1-yl) éthyl mercapto] 1,2,4- triazolo-(4,3a)-pyridine**

Formule I : $R_3 = R_4 = R_5 = H$, $n = 2$

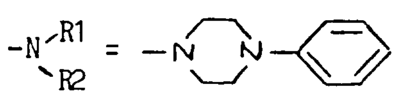

Sous forme de cristaux de point de fusion 100-1 ° C.

Exemple 55 : **3-[(1-benzoyl pipéridin-4-yl) mercapto]-1,2,4-triazolo- (4,3a)-pyridine**

Formule I : $R_3 = R_4 = R_5 = H$, $n = 3$

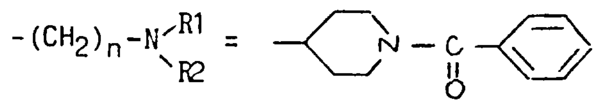

Sous forme de cristaux de point de fusion 132-3 ° C.

Exemple 56 : **3-[2-(4-oxo pipéridin-1-yl) éthyl mercapto]-1,2,4-triazolo-(4,3a)-pyridine**

Formule I : $R_3 = R_4 = R_5 = H$, $n = 2$

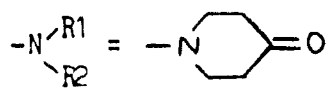

6.5 g de 3-[2-(4-(1,4-dioxa-8-azaspiro-(4,5)-decan-8-yl) éthyl mercapto] 1,2,4-triazolo-(4,3a)-pyridine préparés à l'exemple 53 sont portés à reflux durant 10 heures dans une solution de 200 ml d'acide chlorhydrique 0.5 N. Le mélange réactionnel est ensuite refroidi et basifié avec une solution de bicarbonate de sodium et extrait au chloroforme. La phase chloroformique séchée sur sulfate de magnésium est concentrée sous vide. Le résidu obtenu cristallise dans l'acétate d'isopropyle. Les cristaux sont essorés et séchés. On obtient ainsi 3.5 g de 3-[2-(4-oxo pipéridin-1-yl)ethyl mercapto]-1,2,4-triazolo-(4,3a)-pyridine sous forme de cristaux de point de fusion 76-7 ° C.

Exemple 57 : **1-[3-(4-(3-trifluorométhyl phényl) pipérazin-1-yl) propyl mercapto]-s-triazolo-(4,3a)-quinoline**

Formule I : $R_3 = H$, $R_4-R_5 = 5,6-CH=CH-CH=CH-$, $n = 3$

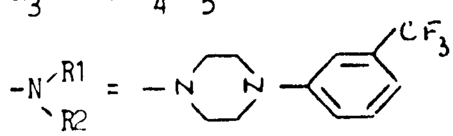

Une suspension de 13.4 g de 1-mercapto-s-triazolo-(4,3a)-quinoline, 20.5 g de 1-chloro 3-[4-(3-trifluoro méthyl phényl) pipérazin-1-yl] propane base et 11.2 ml de triéthylamine dans 150 ml d'éthanol est portée 7 heures à reflux. Après refroidissement le milieu est concentré sous vide, puis le résidu repris dans du chloroforme. La phase organique est lavée à l'eau, séchée sur sulfate de sodium, puis concentrée. Le résidu épais est cristallisé par addition d'éther isopropylique, essoré, séché. On obtient après recristallisation dans l'éthanol 12.7 g de 1-[3-(4- (3-trifluoro méthyl phényl) pipérazin-1-yl) propyl mercapto]-s-triazolo-(4,3a)-quinoline sous forme de cristaux de point de fusion 117-8 ° C.

Exemple 58 : **1-[2-(4-(3-trifluorométhyl phényl) pipérazin-1-yl) éthyl mercapto]-s-triazolo-(4,3a)-quinoline**

Formule I : $R_3 = H$, $R_4-R_5 = 5,6-CH=CH-CH=CH-$, $n = 2$

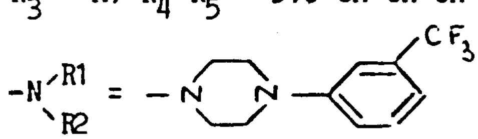

Selon le mode opératoire de l'exemple 57, à partir de 21 g de 1-chloro 2-[4-(3-trifluoro méthyl phényl) pipérazin-1-yl] éthane base et 14.4 g de 1- mercapto-s-triazolo-(4,3a)-quinoline on obtient après recristallisation dans l'éthanol 13.5 g de 1-[2-(4-(3-trifluoro méthyl phényl) pipérazin-1-yl) éthyl mercapto]-s-triazolo-(4,3a)-quinoline sous forme de cristaux de point de fusion 132 ° C.

Exemple 59 : **1-[3-(4-(3-trifluorométhyl phényl) pipérazin-1-yl) propylmercapto] 4-méthyl-s-triazolo (4,3a) quinoline**

Formule I : $R_3$ = 8-$CH_3$, $R_4$-$R_5$ = 5,6-CH=CH-CH=CH-

n = 3 , 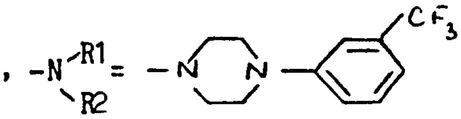

Selon le mode opératoire de l'exemple 57, à partir de 22 g de 1-chloro 3-[4-(3-trifluoro méthyl phényl) pipérazin-1-yl] propane base et 15.4 g de 1-mercapto 4-méthyl-s-triazolo-(4,3a)-quinoline préparé à l'exemple 22, on obtient après recristallisation dans l'acétate d'éthyle 14 g de 1-[3-(4-(trifluoro méthyl phényl) pipérazin-1-yl) propyl mercapto]-4-méthyl-s-triazolo (4,3a)-quinoline sous forme de cristaux de point de fusion 137 ° C.

Exemple 60 : **1-[2-(4-(2,4-difluoro phényl) pipérazin-1-yl) éthylmercapto]-4-méthyl-s-triazolo-(4,3a)-quinoline**

Formule I : $R_3$ = 8-$CH_3$, $R_4$-$R_5$ = 5,6-CH=CH-CH=CH-

n = 2, 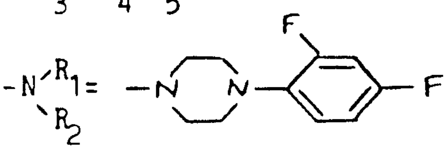

Selon le mode opératoire de l'exemple 57, à partir de 23 g de 1-chloro 2-[4-(2,4-difluoro phényl) pipérazin-1-yl] éthane base préparé à partir du chlorhydrate de l'exemple 7 et 19 g de 1-mercapto 4-méthyl-s-triazolo- (4,3a)-quinoline préparé à l'exemple 22, on obtient après recristallisation dans l'acétate d'éthyle 22 g de 1-[2-(4-(2,4-difluoro phényl) pipérazin-1- yl) éthyl mercapto]-4-méthyl-s-triazolo-(4,3a)-quinoline sous forme de cristaux de point de fusion 124 ° C.

Exemple 61 : **1-[2-(4-(2,4-difluoro phényl) pipérazin-1-yl) éthyl mercapto] -s-triazolo-(4,3a)-quinoline**

Formule I : $R_3$ = H, $R_4$-$R_5$ = 5,6-CH=CH-CH=CH-, n = 2

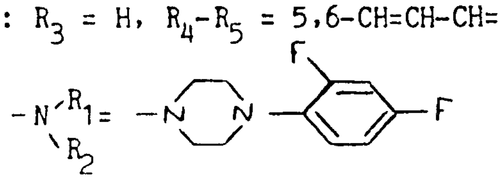

Selon le mode opératoire de l'exemple 57, à partir de 23 g de 1-chloro 2-[4-(2,4-difluoro phényl) pipérazin-1-yl] éthane base préparé à partir du chlorhydrate de l'exemple 7 et 17.8 g de 1-mercapto-s-triazolo-(4,3a)- quinoline on obtient après recristallisation dans l'acétate d'éthyle 13.6 g de 1-[2-(4-(2,4-di fluoro phényl) pipérazin-1-yl) éthyl mercapto]-s-triazolo-(4,3a)-quinoline sous forme de cristaux de point de fusion 114 ° C.

Exemple 62 : **1-[2-(4-(2,4-difluorophényl) pipérazin-1-yl) éthyl mercapto] -5-méthyl-s-triazolo-(4,3a) quinoline**

**Formule I** : $R_3$ = 7-$CH_3$, $R_4$-$R_5$ = 5,6-CH=CH-CH=CH-

n = 2 , 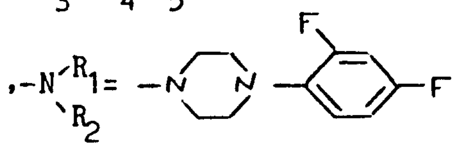

Selon le mode opératoire de l'exemple 57, à partir de 8.4 g de 1-chloro 2-[4-(2,4-difluoro phényl) pipérazin-1-yl] éthane base préparé à partir du chlorhydrate de l'exemple 7 et 7 g de 1-mercapto 5-méthyl-s-triazolo-(4,3a)- quinoline on obtient après recristallisation dans l'acétate d'éthyle 4.7 g de 1-[2-(4-(2,4-difluoro phényl) pipérazin-1-yl) éthyl mercapto]-5-méthyl-s-triazolo-(4,3a)-quinoline sous forme de cristaux de point de fusion 133 ° C.

Exemple 63 : **1-[2-(4-(3-trifluoro méthyl phényl) pipérazin 1-yl) éthyl mercapto]-4-méthyl-s-triazolo-(4,3a) quinoline**

**Formule I** : $R_3$ = 8-$CH_3$, $R_4$-$R_5$ = 5,6-CH=CH-CH=CH-

n = 2, 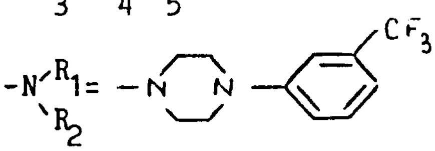

Selon le mode opératoire de l'exemple 57, à partir de 23.3 g de 1-chloro 2-[4-(3-trifluoro méthyl phényl) pipérazin-1-yl] éthane base et 17.2 g de 1-mercapto 4-méthyl-s-triazolo-(4,3a) quinoline préparé à l'exemple 22 on obtient après recristallisation dans l'acétate d'éthyle 16.5 g de 1-[2-(4- (3-trifluoro méthyl phényl) pipérazin-1-yl) éthyl mercapto]-4-méthyl-s-triazolo-(4,3a)-quinoline sous forme de cristaux de point de fusion 120 ° C.

D'une façon identique, selon le mode opératoire de l'exemple 57 on a obtenu :

Exemple 64 : **1-[2-(4-(4-fluoro phényl)-1,2,3,6-tétrahydro pyridin-1-yl) éthyl mercapto]-s-triazolo-(4,3a) quinoline**

**Formule I** : $R_3$ = H, $R_4$-$R_5$ = 5,6-CH=CH-CH=CH-, n = 2

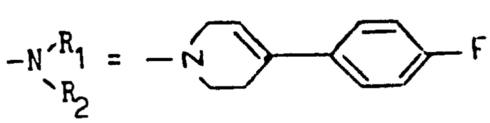

Sous forme de cristaux de point de fusion 130 ° C.

Exemple 65 : **1-[3-(4-(2,4-difluoro phényl)-pipérazin-1-yl) propyl mercaptol -s-triazolo-(4,3a)-quinoline**

**Formule I :** $R_3$ = H, $R_4$-$R_5$ = 5,6-CH=CH-CH=CH-, n = 3

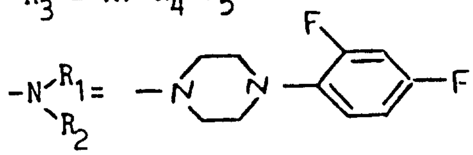

Sous forme de cristaux de point de fusion 131-3 ° C.

Exemple 66 : **1-[3-(4-(2,4-difluoro phényl) pipérazin-1-yl) propyl mercaptol -4-méthyl-s-triazolo-(4,3a) quinoline**

**Formule I :** $R_3$ = 8-$CH_3$, $R_4$-$R_5$ = 5,6-CH=CH-CH=CH-

n = 3, 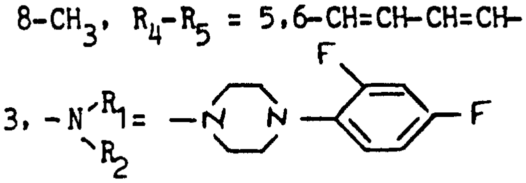

Sous forme de cristaux de point de fusion 137-8 ° C.

Exemple 67 : **1-[3-(4-(2,4-difluoro phényl)-pipérazin-1-yl) propyl mercaptol -4,6,8-triméthyl-s-triazolo (4,3a)-quinoline**

**Formule I :** $R_3$ = 8-$CH_3$, $R_4$-$R_5$ = 6,5-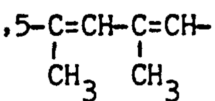

n = 3 , 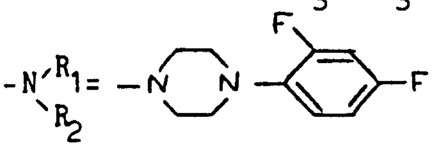

Sous forme de cristaux de point de fusion 128-130 ° C.

Exemple 68 : **Chlorhydrate de 3-[2-(méthylamino) éthylmercaptol 1,2,4-triazolo-(4,3a)-pyridine**

**Formule I** : $R_1$ = $CH_3$, $R_2$ = $R_3$ = R4 = R5 = H, n = 2

Une solution de 18 g de N-méthyl 2-chloro éthylamine chlorhydrate, 20 g de 3-mercapto-(1,2,4)-triazolo-(4,3a)-pyridine et 40 ml de triéthylamine dans 150 ml d'éthanol est portée 2 heures à reflux. Après évaporation, le résidu est repris dans de l'eau, additionné de soude 0.1 N et extrait au chloroforme.
La phase chloroformique est lavée à l'eau, séchée sur sulfate de sodium et évaporée à sec. Le résidu obtenu est dissous dans un mélange éther éthylique-acétone et la solution acidifiée par addition d'une solution d'éther éthylique saturée par de l'acide chlorhydrique gazeux. Le précipité formé est essoré, lavé à l'éther, séché. On obtient 12.6 g de chlorhydrate de 3-[2-(méthyl amino) éthyl mercapto]-1,2,4-triazolo-(4,3a)-pyridine sous forme de cristaux de point de fusion 195-7 ° C.

Selon un mode opératoire identique, les composés suivants ont été synthétisés :

Exemple 69 : **Dichlorhydrate de 3-[2-(4-morpholino) éthyl mercapto]-1,2,4- triazolo-(4,3a)-pyridine**

**Formule I** : $R_3 = R_4 = R_5 = H$, $n = 2$

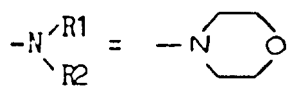

Sous forme de cristaux de point de fusion 205-210 ° C.

Exemple 70 : **Maléate de 3-[(4-benzyl morpholin-3-yl) méthyl mercapto]- 1,2,4- triazolo-(4,3a)-pyridine**

**Formule I** : $R_3 = R_4 = R_5 = H$, $n = 2$

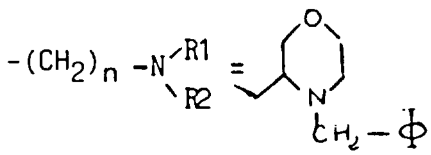

Sous forme de cristaux de point de fusion 159-160 ° C.

Exemple 71 : **Dichlorhydrate de 3-[(4- benzyl morpholin-2-yl) méthyl mercaptol- 1,2,4-triazolo-(4,3a)-pyridine**

**Formule I** : $R_3 = R_4 = R_5 = H$, $n = 3$

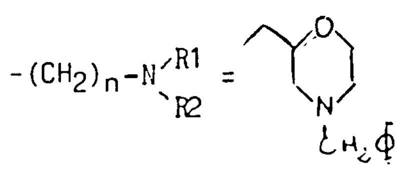

Sous forme de cristaux de point de fusion 203-205 ° C.

Exemple 72 : **2-[-1,2,4-triazolo-(4,3a)-pyridin-3-yl mercapto] éthanol**

**Formule VI** : $R_3 = R_4 = R_5 = H$, $n = 2$

60 g de 3-mercapto-1,2,4-triazolo-(4,3a)-pyridine sont mis en solution dans 500 ml d'éthanol. On ajoute alors 21g de carbonate de sodium et 29 ml de 2-bromo éthanol. Le mélange réactionnel est porté 9 heures au reflux, on filtre les minéraux puis le filtrat est concentré sous vide, repris à l'eau acidulée par de l'acide chlorhydrique 0.5 N et extrait au chloroforme. La phase aqueuse est ensuite basifiée à l'ammoniaque et saturée au chlorure de sodium puis extraite au chloroforme. Cette phase chloroformique obtenue est séchée sur sulfate de magnésium puis concentrée sous vide. Les cristaux formés alors sont repris au pentane, essorés, lavés au pentane et séchés. On obtient ainsi 69.2 g de 2-[-1,2,4-triazolo-(4,3a)-pyridin-3-yl mercapto] éthanol sous forme de cristaux de point de fusion 84 ° C.

Exemple 73 : **1-chloro-2-[-1,2,4-triazolo-(4,3a)-pyridin-3-yl mercapto] éthane**

**Formule IV :** $R_3 = R_4 = R_5 = H$, $X' = Cl$

A 57.9 g de 2-[-1,2,4-triazolo-(4,3a)-pyridin-3-yl mercapto] éthanol préparés à l'exemple 72 en solution dans 400 ml de chloroforme, on ajoute goutte à goutte 53.7 ml de chlorure de thionyle. Le milieu

réactionnel est porté au reflux durant 4 heures puis refroidi. Les cristaux formés sont essorés, lavés au chloroforme puis repris à l'eau. La phase aqueuse est basifiée à l'ammoniaque, puis extraite au chloroforme. La phase chloro- formique, séchée sur sulfate de sodium est concentrée sous vide. Le résidu obtenu cristallise. On récupère ainsi 46.8 g de 1-chloro-2-[-1,2,4- triazolo-(4,3a)-pyridin-3-yl mercaptol éthane sous forme de cristaux de point de fusion 94 ° C.

Exemple 74 : **3-[2-(4-(3-cyanopyridin-2-yl) pipérazin-1-yl) éthyl mercapto]- 1,2,4-triazolo-(4,3a)-pyridine**

Formule I : $R_3 = R_4 = R_5 = H$, $n = 2$,

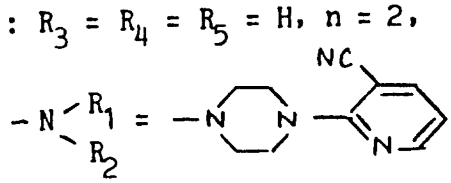

Une solution de 13 g de 1-chloro-2-[1,2,4-triazolo-(4,3a) pyridin-3-yl mercapto] éthane préparés à l'exemple 73 et 11.5 g de (3-cyano pyridin-2-yl) pipérazine dans 200 ml de xylène contenant 0.5 g d'iodure de sodium et 8.5 ml de triéthylamine est portée 7 heures au reflux. Le milieu réactionnel est ensuite refroidi puis la phase xylénique est lavée à l'eau puis extraite avec une solution d'acide chlorhydrique diluée. La phase aqueuse est extraite au chloroforme puis basifiée à froid avec une solution d'ammoniaque. Les produits basiques sont alors extraits au chloroforme qu'on lave à l'eau, sèche sur sulfate de magnésium et qu'on concentre sous vide. Le résidu obtenu est filtré sur gel de silice (éluant: chloroforme-méthanol / 98-2). On obtient ainsi une huile qui cristallise dans l'éther éthylique. Les cristaux essorés et séchés, on récupère 5.5 g de 3-[2-(4-(3-cyanopyridin 2-yl) piperazin-1-yl) éthyl mercapto]-1,2,4-triazolo (4,3a)-pyridine de point de fusion 116-7 ° C.

Selon ce mode opératoire, les composés suivants ont été obtenus :

Exemple 75 : **3-[2-(4-(3-méthoxyphényl) pipérazin-1-yl) éthyl mercapto]- 1,2,4-triazolo-(4,3a)-pyridine**

Formule I : $R_3 = R_4 = R_5 = H$, $n = 2$,

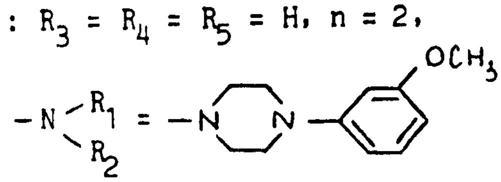

Sous forme de cristaux de point de fusion 105 ° C.

Exemple 76 : **3-[2-(4-(3,4-dichloro phényl) pipérazin-1-yl) éthyl mercapto]- 1,2,4-triazolo-(4,3a)-pyridine**

Formule I : $R_3 = R_4 = R_5 = H$, $n = 2$,

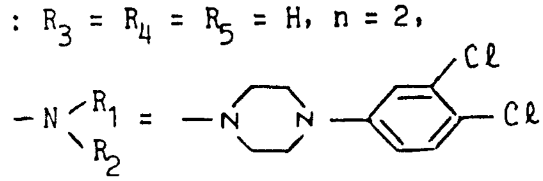

Sous forme de cristaux de point de fusion 122-3 ° C.

Exemple 77 : **3-[2-(4-(3-hydroxy phényl) pipérazin-1-yl) éthyl mercapto]- 1,2,4-triazolo-(4,3a)-pyridine**

Formule I : $R_3 = R_4 = R_5 = H$, $n = 2$,

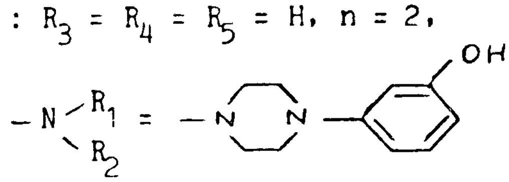

Sous forme de cristaux de point de fusion 152 °C.

Exemple 78 : **3-[2-(4-(4-méthylphénylsulfonyl) pipérazin-1-yl) éthyl mercapto]-1,2,4-triazolo-(4,3a)-pyridine**

Formule I : $R_3 = R_4 = R_5 = H$, $n = 2$,

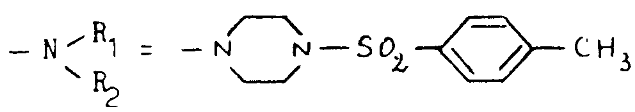

A 32.7 g de 3-mercapto-1,2,4-triazolo-(4,3a)-pyridine en solution dans 300 ml d'éthanol, on ajoute 30 g de carbonate de potassium et on chauffe à reflux. Après 15 minutes, on ajoute 65.5 g de 1-chloro-2[4-(4-méthylphényl sulfonyl) pipérazin-1-yl] éthane en solution dans 100 ml d'éthanol et le reflux est poursuivi 3 heures. Le mélange réactionnel est ensuite refroidi, les minéraux sont filtrés et le filtrat est concentré sous vide. Le résidu est repris au chloroforme qu'on lave à l'eau puis la phase chloroformique est extraite avec une solution d'acide chlorhydrique 0.5 N. La phase aqueuse acide est ensuite basifiée à froid avec une solution aqueuse de soude 0.5 N puis extraite au chloroforme. La phase chloroformique est lavée à l'eau, séchée sur sulfate de sodium puis concentrée sous vide pour donner 73 g de 3-[2-(4- (4-méthylphénylsulfonyl) pipérazin-1-yl) éthyl mercapto] 1,2,4-triazolo- (4,3a)-pyridine sous forme d'huile épaisse qu'on utilise brute pour l'étape suivante.

Exemple 79 : **3-[2-(pipérazin-1-yl) éthylmercapto]1,2,4,-triazolo (4,3a)- pyridine**

Formule I : $R_3 = R_4 = R_5 = H$, $n = 2$,

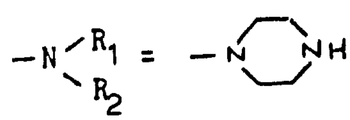

73 g de 3-[2-(4-(4-méthylphénylsulfonyl) pipérazin-1-yl) éthyl mercapto] -1,2,4-triazolo-(4,3a)-pyridine préparés à l'exemple 78 sont mis en solution dans 360 ml d'une solution d'acide chlorhydrique concentré et porté 8 heures au reflux. Après refroidissement la solution est neutralisée par de la soude et extraite au chloroforme. La phase chloroformique est séchée sur sulfate de magnésium et concentrée sous vide pour donner 37.4 g de 3-[2-(pipérazin- 1-yl) éthyl mercapto]-1,2,4-triazolo (4,3a)-pyridine sous forme d'une huile qui cristallise dans l'acétone F = 104 °C.

Exemple 80 : **3-[2-(4-(pyrimidin-2-yl) pipérazin-1-yl) éthyl mercapto] 1,2,4- triazolo-(4,3a)-pyridine**

**Formule I :** $R_3 = R_4 = R_5 = H$, $n = 2$,

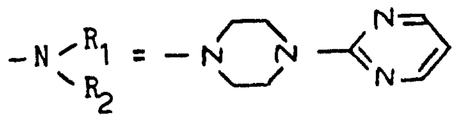

4.8 g de 3-[2-(pipérazin-1-yl) éthylmercapto]-1,2,4-triazolo-(4,3a)-pyridine préparés à l'exemple 79 et 2.1 g de 2-chloro pyrimidine sont portés à reflux dans 140 ml d'isopropanol en présence de 2.5 g de carbonate de potassium. Après 2 heures de chauffage, le milieu est refroidi, les minéraux filtrés et le filtrat concentré. Le résidu huileux est filtré sur gel de silice (éluant: chloroforme-methanol/90-10). Le résidu obtenu alors cristallise dans un mélange éther éthylique-éther isopropylique pour donner 2.8 g de 3- [2-(4-(pyrimidin-2-yl) pipérazin-1-yl) éthyl mercapto]-1,2,4-triazolo-(4,3a) pyridine sous forme de cristaux de point de fusion 126-7 ° C.

Selon ce mode opératoire, les composés suivants ont été obtenus :

Exemple 81 : **3-[2-(4-(3-trifluorométhyl pyridin-2-yl) pipérazin-1-yl) éthyl mercapto]-1,2,4- triazolo-(4,3a)-pyridine**

**Formule I :** $R_3 = R_4 = R_5 = H$, $n = 2$,

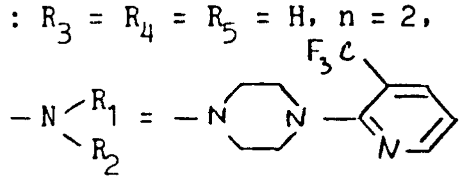

Sous forme de cristaux de point de fusion 67-8 ° C.

Exemple 82 : **3-[2-(4-(4-trichlorométhyl pyrimidin-2-yl) pipérazin-1-yl) éthyl mercapto]-1,2,4-triazolo (4,3a)-pyridine**

**Formule I :** $R_3 = R_4 = R_5 = H$, $n = 2$,

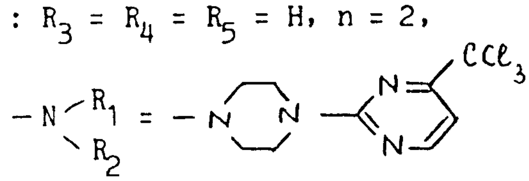

Sous forme de cristaux de point de fusion 132 ° C.

Exemple 83 : **3-[2-(4-(5-cyanopyridin-2-yl) pipérazin-1-yl) éthyl mercapto]- 1,2,4-triazolo-(4,3a)-pyridine**

**Formule I :** $R_3 = R_4 = R_5 = H$, $n = 2$,

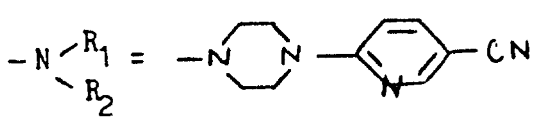

Sous forme de cristaux de point de fusion 134 ° C.

<u>Exemple 84</u> : **3-[2-(4-(4-methyl pyrimidin-2-yl) piperazin-1-yl) éthyl mercapto]-1,2,4-triazolo-(4,3a)-pyridine**

Formule I : $R_3 = R_4 = R_5 = H$, $n = 2$,

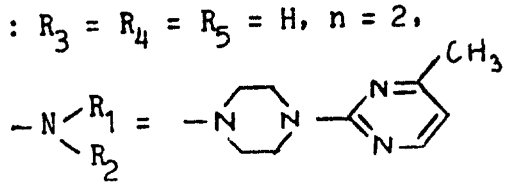

sous forme de cristaux de point de fusion 129 ° C.

<u>Exemple 85</u> : **3-[2-(4-(3-nitropyridin-2-yl) pipérazin-1-yl) éthyl mercapto]- 1,2,4-triazolo-(4,3a)-pyridine**

Formule I : $R_3 = R_4 = R_5 = H$, $n = 2$,

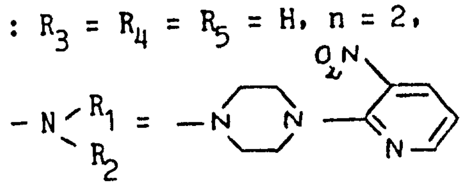

Sous forme de cristaux de point de fusion 166 ° C.

<u>Exemple 86</u> : **3-[2-(4-(3-aminopyridin-2-yl) pipérazin-1-yl) éthyl mercapto]- 1,2,4-triazolo-(4,3a)-pyridine**

Formule I : $R_3 = R_4 = R_5 = H$, $n = 2$,

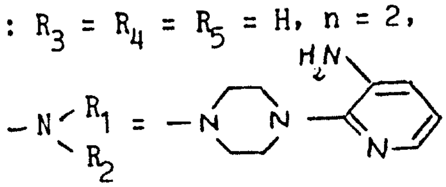

3 g de 3-[2-(4-(3-nitropyridin-2-yl) pipérazin-1-yl) éthyl mercapto]- 1,2,4-triazolo-(4,3a)-pyridine préparés à l'exemple 85 sont dissous dans 50 ml d'éthanol et hydrogénés à température ambiante et pression normale en présence de Ni/Raney. Quand la quantité théorique d'hydrogène est absorbée, le nickel est filtré et le filtrat concentré sous vide. Le résidu est filtré sur gel de silice (éluant : chloroform-méthanol/96-4). Le résidu récupéré cristallise dans l'éther isopropylique. On récupère ainsi 1.8 g de cristaux de 3-[2-(4-(3-amino pyridin-2-yl) pipérazin-1-yl) éthyl mercapto]-1,2,4- triazolo-(4,3a)-pyridine de point de fusion 104 ° C.

<u>Exemple 87</u> : **Maléate de 3-[(pipéridin-4-yl) mercapto]-1,2,4-triazolo (4,3a)-pyridine**

Formule I : $R_3 = R_4 = R_5 = H$, $n = 3$,

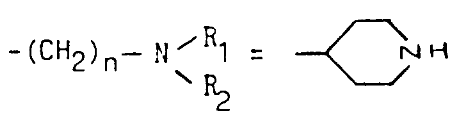

6.3 g de 3-[(1-benzoyl pipéridin-4-yl) mercapto] 1,2,4-triazolo (4,3a) pyridine préparés à l'exemple 55 en solution dans 60 ml d'une solution d'acide chlorhydrique 1.55 N sont portés 7 heures à reflux. Après refroidissement, l'acide benzoïque formé est essoré, le filtrat extrait au chloroforme puis concentré à moitié. La solution aqueuse est basifiée au carbonate de sodium puis extraite au chloroforme. La phase organique est séchée puis concentrée pour donner 4.5 g d'huile.

Cette huile est reprise dans l'acétone et traitée avec une solution acétonique de 2.3 g d'acide maléique.

On obtient ainsi 3.3 g de maléate de 3-[(pipéridin-4-yl) mercapto] -1,2,4-triazolo-(4,3a)- pyridine sous forme de cristaux de point de fusion 150-1 ° C.

Exemple 88 : **3-[2-(4-(2,5-difluoro phényl) pipérazin-1-yl) éthyl mercapto]- 1,2,4-triazolo-(4,3a)-pyridine**

**Formule I :** $R_3 = R_4 = R_5 = H$, $n = 2$,

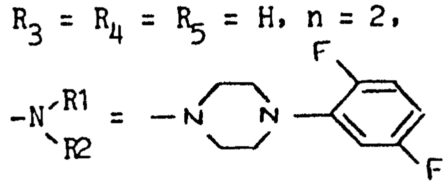

Selon le mode opératoire de l'exemple 28, mais à partir de 7.5 g de 3-mercapto-1,2,4-triazolo-(4,3a)-pyridine et 12.9 g de 1-chloro 2-[4-(2,5-difluoro phenyl) piperazin 1-yl] ethane base, obtenu à partir du dichlorhydrate préparé à l'exemple 10, on obtient 3.3 g de 3-[2-(4-(2,5- difluoro phényl) pipérazin-1-yl] éthyl mercapto]-1,2,4-triazolo-(4,3a)- pyridine sous forme de cristaux de point de fusion 83-84 ° C.

**PHARMACOLOGIE**

*** Principe**

L'activité analgésique des produits des exemples revendiqués dans la présente demande a été évaluée selon 2 méthodologies :

- la méthode de Siegmund et al. (1) où la réaction douloureuse est provoquée par la phénylbenzoquinone. Cette méthode permet d'apprécier l'activité analgésique de molécules agissant aussi bien à la périphérie qu'au niveau central.
- la méthode de la plaque chauffante décrite par Eddy et al. (2).
  Cette méthode est plus spécifique des molécules possédant une activité analgésique d'origine centrale.

*** Matériel**

Animaux

Des souris mâles de souche $CD_1$ (Charles River) et $OF_1$ (Iffa Credo) pesant de 19 à 22 g ont été respectivement utilisées pour le test de Siegmund et le test d'Eddy. Les tests ont été effectués à la température ambiante de 21 ± 2 ° C.

Produits (exemples revendiqués)

Les produits ont été mis en suspension dans un mélange aqueux de gomme arabique à 1 %, de chlorure de sodium à 0.1 % et de Tween 80 à 0.001 %.

Les animaux des groupes témoins ont reçu le véhicule.

*** Méthodes**

**. Test de Siegmund à la phénylbenzoquinone**

Mode opératoire

Les souris reçoivent par voie intrapéritonéale 0.20 à 0.22 ml d'une solution hydro-alcoolique de phénylbenzoquinone à 0.02 %, une heure après l'administration du produit étudié.

Le nombre de réactions douloureuses (torsions et étirements) est compté de la 5e à la 10e minute.

Schéma d'administration

Les produits des exemples étudiés ont été administrés à des lots de 6 à 12 souris par tubage gastrique (0.5 ml.20 $g^{-1}$) une heure avant la phénylbenzoquinone, selon le schéma de doses suivant :

0, 0.3, 1, 3, 10, 30, 100 $mg.kg^{-1}$.

Expression des résultats

Le pourcentage d'inhibition des manifestations douloureuses a été calculé à partir des moyennes de torsions par lot.

**. Test de la plaque chauffante (Eddy)**

Mode opératoire

Les souris sont placées sur une plaque métallique thermostatée à 56 °C. Le temps de réaction des animaux, objectivé par le léchage des pattes antérieures, est mesuré. Dans tous les cas, les souris ne sont pas laissées en contact avec la plaque chauffante plus de 30 secondes.

Schéma d'administration

Des lots de 10 souris reçoivent, par voie intrapéritonéale (0.5 ml.20 $g^{-1}$), les produits des exemples étudiés, 30 minutes avant d'être placées sur la plaque. Ces produits sont administrés selon le schéma de doses suivant :

0, 3, 10, 30, 100 $mg.kg^{-1}$.

Expression des résultats

La différence entre le temps maximum de réaction (30 secondes) et la moyenne des temps de réaction pour chacun des groupes traités ont été calculées.

*** Statistiques**

Les doses actives 50 et leurs limites de confiance ont été déterminées à partir de la régression linéaire effectuée sur les paramètres mesurés.

*** Résultats**

Les tableaux I et II regroupent pour les deux tests utilisés les $DA_{50}$ obtenues.

*** Toxicité**

Des études préliminaires de toxicité réalisées chez le rat par voie orale ont permis de montrer que la plupart des produits des exemples revendiqués étaient bien tolérés pour des doses < à 300 $mg.kg^{-1}$.

Tableau I

| Activité analgésique des produits des exemples revendiqués sur le test de Siegmund à la phénylbenzoquinone après administration par voie orale | | |
|---|---|---|
| Produit de l'exemple | Nombre d'animaux par lot | DA50 et intervalle de confiance ($mg.kg^{-1}$) |
| 1 | 12 | 2.16 (1.28 - 3.66) |
| 24 | 12 | 4.53 (2.60 - 7.90) |
| 28 | 12 | 22.37 (16.05 - 31.19) |
| 31 | 6 | 3.60 (1.26 - 10.32) |
| 32 | 12 | 12.57 (9.05 - 17.46) |
| 33 | 12 | 1.71 (1.00 - 2.92) |
| 41 | 6 | 3.19 (1.38 - 7.34) |
| 43 | 6 | 13.87 (10.38 - 18.52) |
| 44 | 6 | 10.98 (7.21 - 16.70) |
| 45 | 6 | 33.02 (23.97 - 45.5) |
| 48 | 6 | 16.10 (9.71 - 26.67) |
| 49 | 6 | 2.45 (1.06 - 5.66) |
| 57 | 6 | 8.56 (2.15 - 34) |
| 59 | 6 | 14.12 (6.11 - 32.64) |
| 62 | 6 | 11.02 (3.99 - 30.41) |
| 63 | 6 | 8.17 (5.28 - 12.64) |
| 66 | 6 | 17.85 (14.01 - 22.75) |

Tableau II

| Activité analgésique des produits des exemples revendiqués sur le test de la plaque chauffante après administration par voie intrapéritonéale | | |
|---|---|---|
| Produit de l'exemple | Nombre d'animaux par lot | DA50 et intervalle de confiance ($mg.kg^{-1}$) |
| 1 | 10 | 6.52 (2.97 - 14.35) |
| 24 | 10 | 35.07 (22.80 - 53.75) |
| 28 | 10 | 88.55 (56.02 - 139.4) |
| 31 | 10 | > 10 |
| 32 | 10 | > 30 |
| 33 | 10 | 12.87 (8.95 - 18.51) |
| 41 | 10 | 17.7 (15.06 - 20.40) |
| 43 | 10 | non calculable |
| 44 | 10 | 11.27 (6.08 - 20.86) |
| 49 | 10 | > 30 |

Conclusion

En thérapeutique humaine, les composés de formule (I), ou de formule X, et éventuellement leurs sels d'addition non toxiques peuvent être administrés notamment par voie orale sous forme de gélules ou de comprimés dosés de 25 à 200 mg de principe actif, par voie rectale sous forme de suppositoires contenant de 100 à 800 mg de principe actif ou par injection intramusculaire ou intraveineuse sous forme de solutions physiologiquement acceptables renfermant de 5 à 40 mg de principe actif.

Ces différents composés de formule I ou leurs sels d'addition non toxiques possèdent des propriétés antalgiques utilisables avec profit dans le traitement de la douleur d'étiologies diverses..

En outre, la mise en évidence pour ces mêmes dérivés de propriétés particulièrement intéressantes au niveau du système nerveux central, en particulier une activité anxiolytique atypique non sédative, permet

d'espérer un bénéfice indéniable dans le traitement de la composante psychique de la douleur.

Références bibliographiques

(1) Siegmund E., Cadmus R., Lu G.
A method for evaluating both non-narcotic and narcotic analgesics.
Proc. Soc. Exp. Biol. Med., 1957, 95, 729-731.
(2) Eddy N.B., Touchberry C.F., Lieberman J.E.
Synthetic analgesics : 1 - Methadone isomers and derivatives.
J. Pharmacol. Exp. Ther., 1950, 98, 121-137.

TABLEAU III

| Code | Formule | Exemple |
|---|---|---|
| 5221-01 | 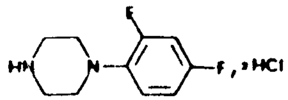 | 1 |
| 5222-01 | 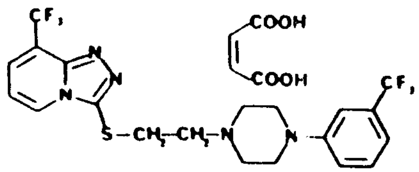 | 24 |
| 26-4 | 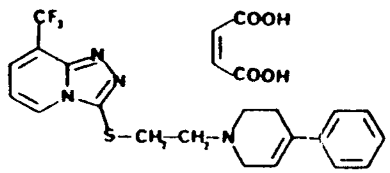 | 25 |
| 26-25 | 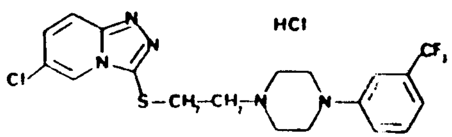 | 26 |
| 26-26 | 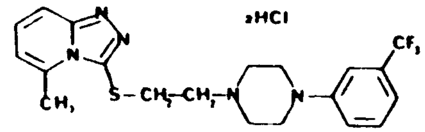 | 27 |
| 5222-02 | 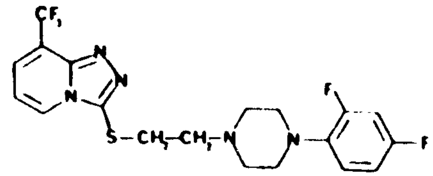 | 28 |
| 26-10 | 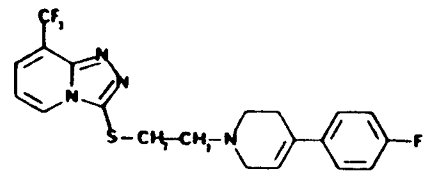 | 29 |
| 26-19 | 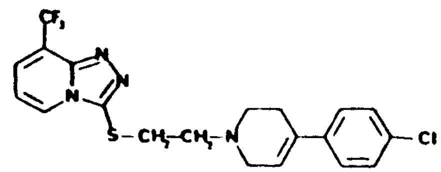 | 30 |
| 26-27 | 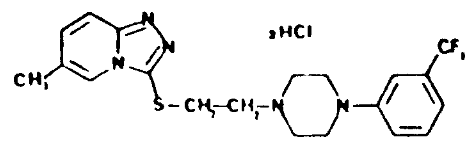 | 31 |

| Code | Formule | Exemple |
|---|---|---|
| 5222-03 | 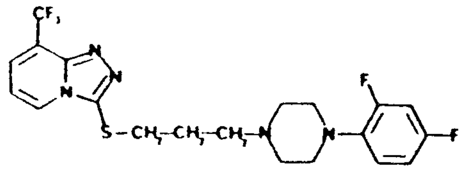 | 32 |
| 5222-04 | 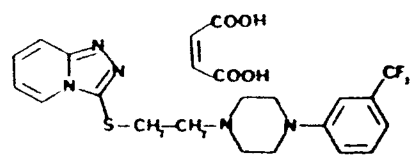 | 33 |
| 26-13 | 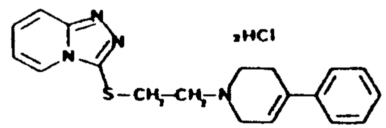 $_2HCl$ | 34 |
| 26-24 | 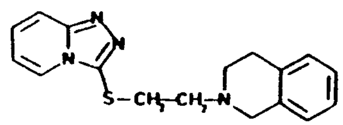 , $_2HCl$ | 35 |
| 26-28 | 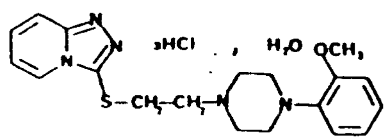 $_2HCl$ , $H_2O$ | 36 |
| 26-29 | 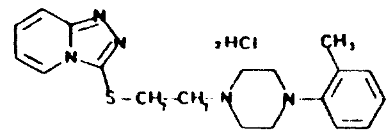 $_2HCl$ | 37 |
| 26-16 | 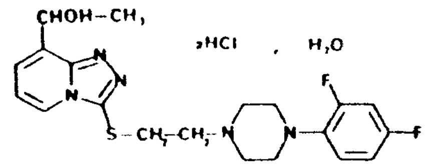 $_2HCl$ , $H_2O$ | 38 |
| 26-31 | 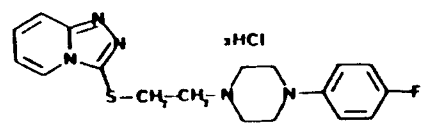 $_2HCl$ | 39 |
| 26-32 | 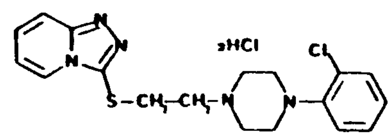 $_2HCl$ | 40 |
| 26-34 | 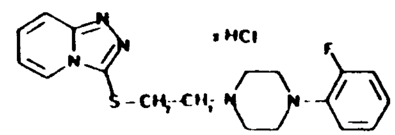 $_2HCl$ | 41 |

| Code | Formule | Exemple |
|---|---|---|
| 26-35 | 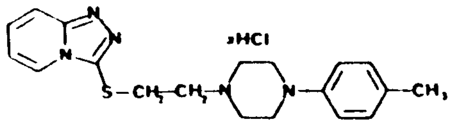 | 42 |
| 5222-05 | 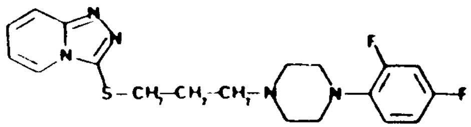 | 43 |
| 5222-06 | 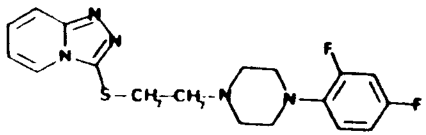 | 44 |
| 26-12 | 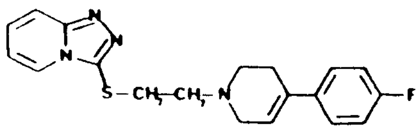 | 45 |
| 26-14 | 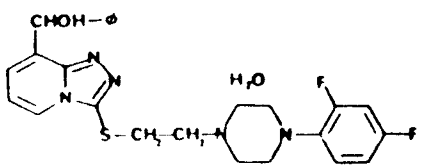 | 46 |

| Code | Formule | Exemple |
|---|---|---|
| 26-17 | 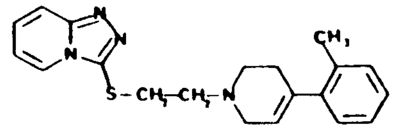 | 47 |
| 26-18 | 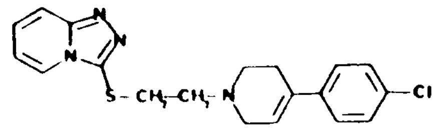 | 48 |
| 26-30 | 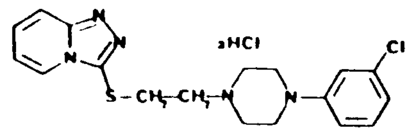 | 49 |
| 26-39 | 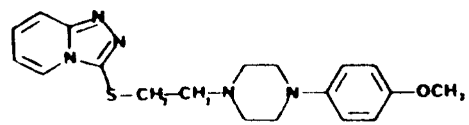 | 50 |
| 26-46 | 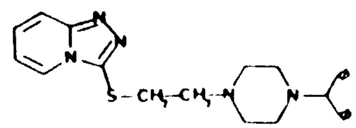 | 51 |

| Code | Formule | Exemple | Code | Formule | Exemple |
|---|---|---|---|---|---|
| 26-49 | 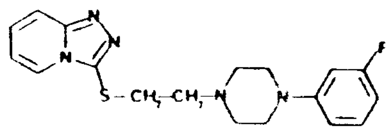 | 53 | 26-3 | 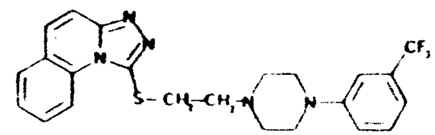 | 58 |
| 26-51 | 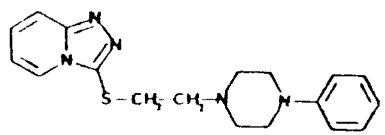 | 54 | 26-5 | 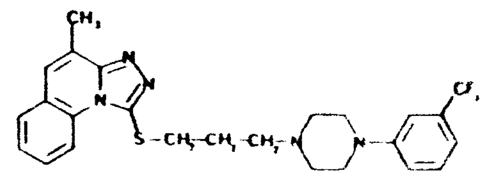 | 59 |
| 26-52 | 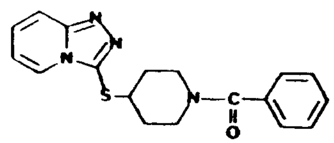 | 55 | 26-6 | 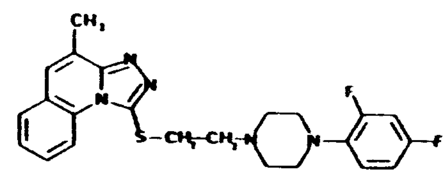 | 60 |
| 26-50 | 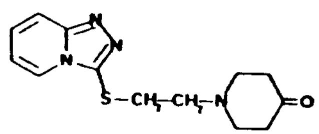 | 56 | 26-7 | 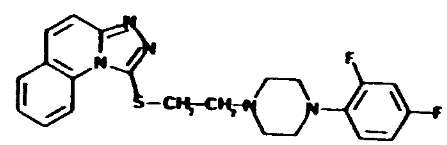 | 61 |
| 26-2 | 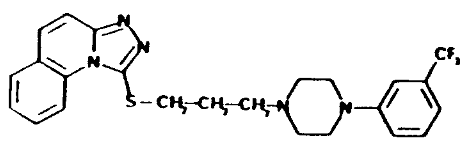 | 57 | 26-8 | 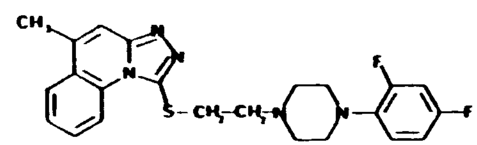 | 62 |

| Code | Formule | Exemple | Code | Formule | Exemple |
|---|---|---|---|---|---|
| 26-9 | 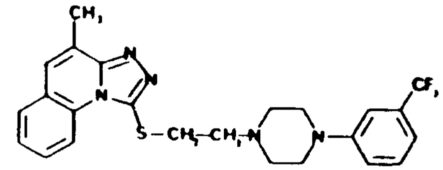 | 63 | 26-15 | 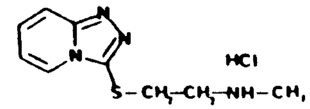 | 68 |
| 26-11 | 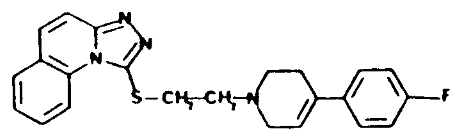 | 64 | 8039-01 | 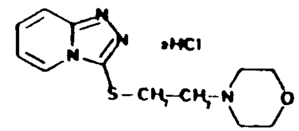 | 69 |
| 26-21 | 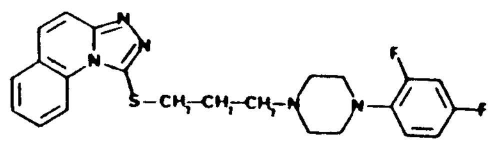 | 65 | 26-1 | 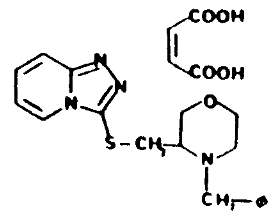 | 70 |
| 26-22 | 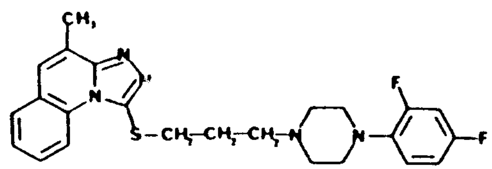 | 66 | 26-20 | 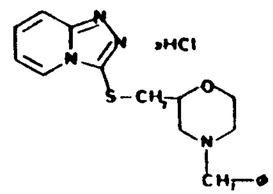 | 71 |
| 26-23 | 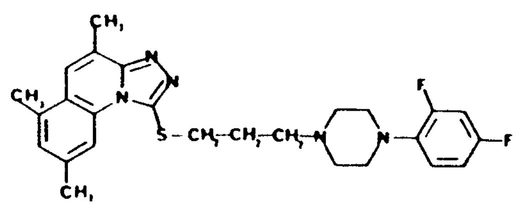 | 67 | 26-33 | 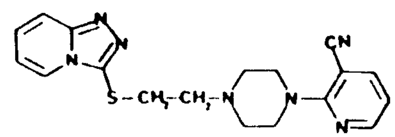 | 74 |

| Code | Formule | Exemple | Code | Formule | Exemple |
|---|---|---|---|---|---|
| 26-38 | 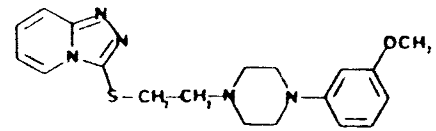 | 75 | 26-43 | 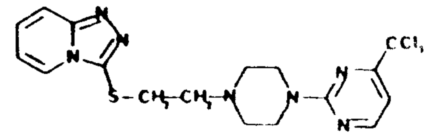 | 82 |
| 26-37 | 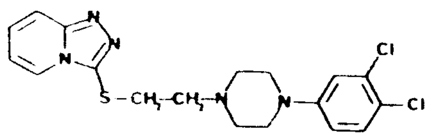 | 76 | 26-44 | 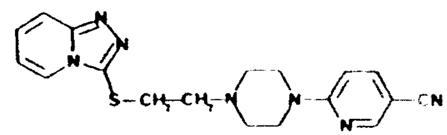 | [illegible] |
| 26-41 | 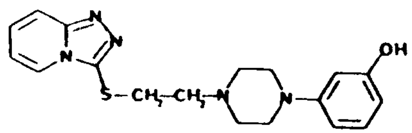 | 77 | 26-45 | 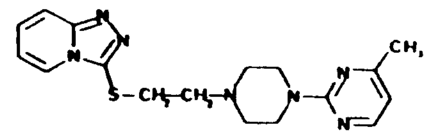 | 84 |
| 26-42 | 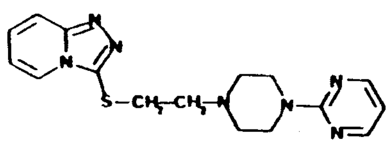 | 80 | 26-47 | 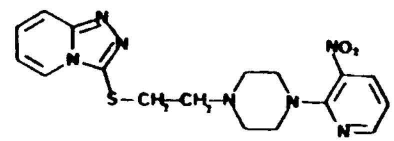 | 85 |
| 26-36 | 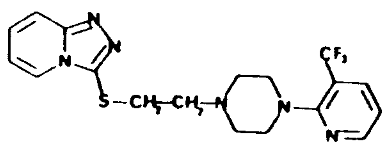 | 81 | 26-48 | 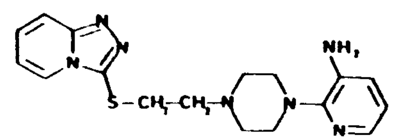 | 86 |

| Exemple | Formule | Code |
| --- | --- | --- |
| 87 | 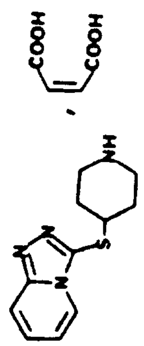 | 26-53 |
| 88 | 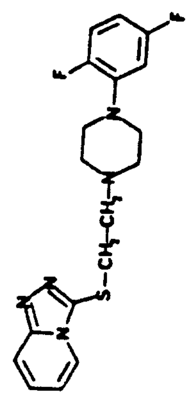 | 26-55 |

**Revendications**

**Revendications pour les Etats contractants suivants : AT, BE, CH, DE, FR, GB, IT, LI, LU, NL, SE**

**1.** Nouveaux composés, caractérisés en ce qu'ils répondent à la formule :

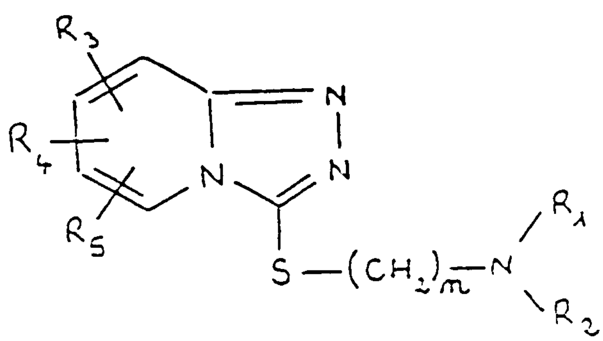

dans laquelle :

- n représente un nombre entier compris entre 1 et 8 ; optimalement 2 ou 3 ; $(CH_2)_n$-N-$R_1$ peut également former un hétérocycle à 6 atomes ;
- $R_1$ et $R_2$ peuvent représenter indépendamment l'hydrogène, un alkyle inférieur de 1 à 5 atomes de carbone, ou former ensemble avec l'atome d'azote un cycle choisi parmi la pipéridine, la morpholine, la phényl tétrahydro pyridine, la pipérazine, la pipérazine N-substituée par un phényle ou un hétérocycle azoté choisi parmi pyridine et pyrimidine ; dans la cas des phényl tétrahydropyridines et des pipérazines substituées par un phényl ou un hétérocycle azoté choisi parmi pyridine et pyrimidine, la phényl ou l'hétérocycle azoté peut être substitué par des halogènes , des groupements méthoxy, hydroxy, nitro, amino, cyano, méthyle, trifluorométhyle et trichlorométhyle ;
- $R_3$, $R_4$ et $R_5$ peuvent représenter indépendamment l'hydrogène, un méthyle, un hydroxy benzyle, un halogène, un trifluorométhyle, ou deux d'entre eux peuvent former un phényle dans le cas des

triazoloquinolines ;

ainsi que les sels d'addition d'acide non toxiques ou un composé choisi parmi :

a) Dichlorhydrate hydraté de 3-[2-(4-(2,4-difluorophényl) pipérazin -1-yl) éthyl mercapto]8-(1-hydroxy éthyl)-1,2,4-triazolo-(4,3a)-pyridine ;

b) 3-[2-(4-(diphényl méthyl) pipérazin-1-yl) éthyl mercapto]-1,2,4-triazolo-(4,3a)-pyridine ; et

c) 3-[(1-benzoyl pipéridin-4-yl) mercapto]-1,2,4-triazolo-(4,3a)-pyridine.

2. Nouveaux composés selon la revendication 1, caractérisés en ce que $R_3 = R_4 = R_5$ = hydrogène.

3. Nouveaux composés selon la revendication 1, caractérisés en ce que $R_4 = R_5$ = hydrogène, $R_3$ = trifluorométhyle.

4. Nouveaux composés selon la revendication 1, 2 ou 3, caractérisés en ce que n = 2 ou 3.

5. Nouveaux composés selon l'une quelconque des revendications 1 à 4, caractérisés en ce que $NR_1R_2$ peut représenter la 4-(3-trifluoro -méthyl phényl) pipérazine.

6. Nouveaux composés selon l'une quelconque des revendications 1 à 4, caractérisés en ce que $NR_1R_2$ peut représenter la 4-(2,4-difluoro phényl) pipérazine.

7. Nouveaux composés selon l'une quelconque des revendications 1 à 4, caractérisés en ce que $NR_1R_2$ peut représenter la 4-(3-chlorophényl) pipérazine.

8. Nouveaux composés selon l'une quelconque des revendications 1 à 4, caractérisés en ce que $NR_1R_2$ peut représenter la 4-(2-fluorophényl) pipérazine.

9. Nouveau composé selon la revendication 1, caractérisé en ce qu'il répond à la formule :

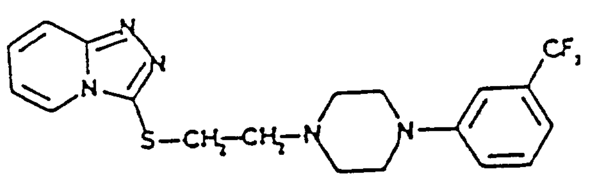

10. Nouveau composé selon la revendication 1, caractérisé en ce qu'il répond à la formule :

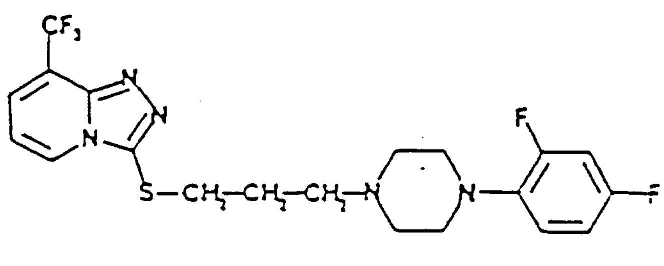

11. Nouveau composé selon la revendication 1, caractérisé en ce qu'il répond à la formule :

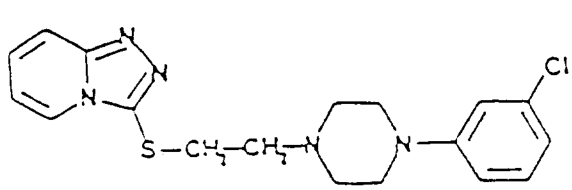

**12.** Nouveau composé selon la revendication 1, caractérisé en ce qu'il répond à la formule :

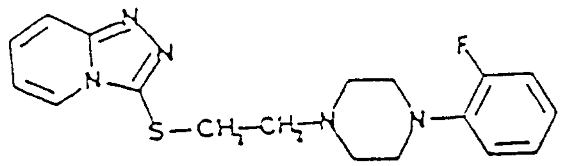

**13.** Procédé de préparation des nouveaux composés selon l'une quelconque des revendications 1 à 12, caractérisé en ce que l'on fait réagir une 3-mercapto-1,2,4,triazolo -(4,3-a) pyridine de formule II suivante :

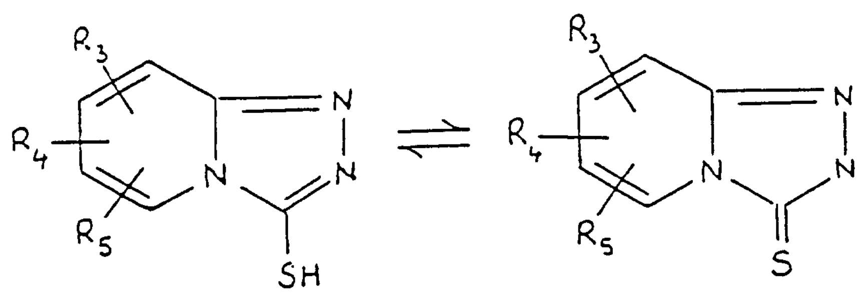

Formule (II)

dans laquelle $R_3$, $R_4$ et $R_5$ sont tels que précédemment définis, avec une halogéno-alkylamine ou une tosyloxyalkylamine ou une mésyloxyalkylamine de formule III suivante :

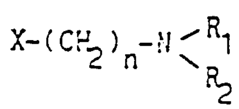

Formule (III)

dans laquelle n, $R_1$ es $R_2$ sont tels que précédemment définis, X étant un halogène ou un bon groupement partant tel que tosyloxy ou mésyloxy, soit par métallation de la fonction thiol par un agent classique de métallation, soit en présence d'une base, dans un solvant organique usuel, à une température comprise entre 20 et 150°C.

**14.** Produit intermédiaire utile pour la préparation de produits de la revendication 1 qui est la 4-(2,4-difluorophényl)-pipérazine ou un de ses sels d'addition.

**15.** Composition pharmaceutique, caractérisée en ce qu'elle comprend à titre d'ingrédient actif au moins un composé selon l'une quelconque des revendications 1 à 12, ou 14, dans un véhicule ou excipient pharmaceutiquement acceptable.

**16.** Composition pharmaceutique, caractérisée en ce qu'elle comprend à titre d'ingrédient actif au moins un composé tel que préparé par le procédé selon la revendication 13, dans un véhicule ou excipient pharmaceutiquement acceptable.

**17.** Médicament doué de propriétés antalgiques, caractérisé en ce qu'il contient au moins un composé selon l'une quelconque des revendications 1 à 12, ou 14.

**18.** Médicament agissant sur le système nerveux central en particulier comme anxiolytique non sédatif, caractérisé en ce qu'il contient au moins un composé selon l'une quelconque des revendications 1 à 12, 14.

**Revendications pour l'Etat contractant suivant : ES**

**1.** Procédé de préparation de nouveaux composés répondant à la formule :

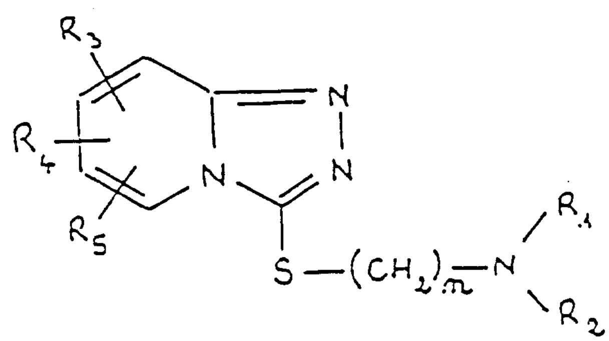

dans laquelle :

- n représente un nombre entier compris entre 1 et 8 ; optimalement 2 ou 3 ; $(CH_2)_n$-N-$R_1$ peut également former un hétérocycle à 6 atomes ;
- $R_1$ et $R_2$ peuvent représenter indépendamment l'hydrogène, un alkyle inférieur de 1 à 5 atomes de carbone, ou former ensemble avec l'atome d'azote un cycle choisi parmi la pipéridine, la morpholine, la phényl tétrahydro pyridine, la pipérazine, la pipérazine N-substituée par un phényle ou un hétérocycle azoté choisi parmi pyridine et pyrimidine ; dans le cas des phényl tétrahydropyridines et des pipérazines substituées par un phényl ou un hétérocycle azoté choisi parmi pyridine et pyrimidine le phényl ou l'hétérocycle azoté peut être substitué par des halogènes , des groupements méthoxy, hydroxy, nitro, amino, cyano, méthyle, trifluorométhyle et trichlorométhyle ;
- $R_3$, $R_4$ et $R_5$ peuvent représenter indépendamment l'hydrogène, un méthyle, un hydroxy benzyle, un halogène, un trifluorométhyle, ou deux d'entre eux peuvent former un phényle dans le cas des triazoloquinolines ;

ainsi que les sels d'addition d'acide non toxiques
ou un composé choisi parmi :

a) Dichlorhydrate hydraté de 3-[2-(4-(2,4-difluorophényl) pipérazin -1-yl) éthyl mercapto]8-(1-hydroxy éthyl)-1,2,4-triazolo-(4,3a)-pyridine ;
b) 3-[2-(4-(diphényl méthyl) piperazin-1-yl) éthyl mercapto]-1,2,4-triazolo-(4,3a)-pyridine ; et
c) 3-[(1-benzoyl pipéridin-4-yl) mercapto]-1,2,4-triazolo-(4,3a)-pyridine.

caractérisé en ce qu'on fait réagir une 3-mercapto-1,2,4-triazolo-(4,3-a)-pyridine de formule II suivante :

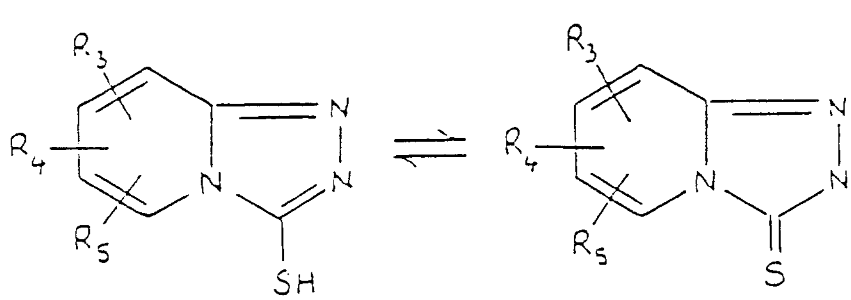

Formule (II)

dans laquelle $R_3$, $R_4$ et $R_5$ sont tels que précédemment définis, avec une halogéno-alkylamine ou une

tosyloxyalkylamine ou une mésyloxyalkylamine de formule III suivante :

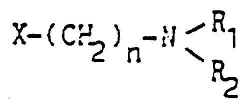

Formule (III)

dans laquelle n, $R_1$ et $R_2$ sont tels que précédemment définis, X étant un halogène ou un bon groupement partant tel que tosyloxy ou mésyloxy, soit par métallation de la fonction thiol par un agent classique de métallation, soit en présence d'une base, dans un solvant organique usuel, à une température comprise entre 20 et 150°C.

**2.** Procédé selon la revendication 1, caractérisé en ce qu'on prépare le composé pour lequel $R_3 = R_4 = R_5$ = hydrogène.

**3.** Procédé selon la revendication 1, caractérisé en ce qu'on prépare le composé pour lequel $R_4 = R_5$ = hydrogène, $R_3$ = trifluorométhyle.

**4.** Procédé selon la revendication 1, 2 ou 3, caractérisé en ce qu'on prépare les composés pour lesquels n = 2 ou 3.

**5.** Procédé selon l'une quelconque des revendications 1 à 4, caractérisé en ce qu'on prépare les composés pour lesquels $NR_1R_2$ peut représenter la 4-(3-trifluoro-méthyl phényl) pipérazine.

**6.** Procédé selon l'une quelconque des revendications 1 à 4, caractérisé en ce qu'on prépare les composés pour lesquels $NR_1R_2$ peut représenter le 4-(2,4-difluoro phényl) pipérazine.

**7.** Procédé selon l'une quelconque des revendications 1 à 4, caractérisé en ce qu'on prépare les composés pour lesquels $NR_1R_2$ peut représenter la 4-(3-chlorophényl) pipérazine.

**8.** Procédé selon l'une quelconque des revendications 1 à 4, caractérisé en ce qu'on prépare les composés pour lesquels $NR_1R_2$ peut représenter la 4-(2-fluorophényl) pipérazine.

**9.** Procédé selon la revendication 1, caractérisé en ce qu'on prépare le composé répondant à la formule :

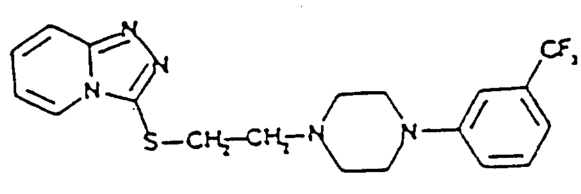

**10.** Procédé selon la revendication 1, caractérisé en ce qu'on prépare le composé répondant à la formule :

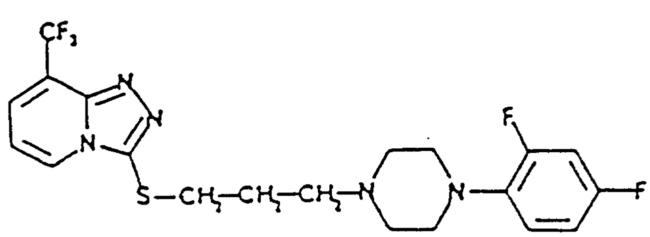

**11.** Procédé selon la revendication 1, caractérisé en ce qu'on prépare le composé répondant à la formule :

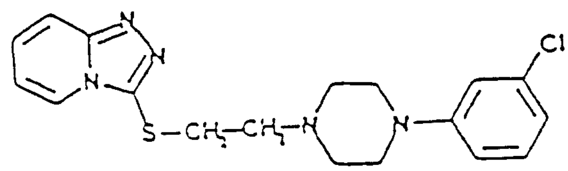

**12.** Procédé selon la revendication 1, caractérisé en ce qu'on prépare le composé répondant à la formule :

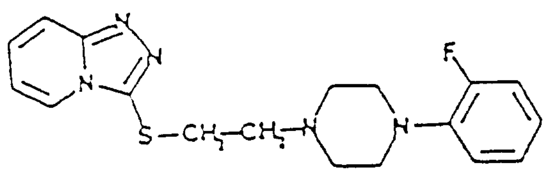

**13.** Procédé de préparation des composés selon l'une quelconque des revendications 1 à 12, caractérisé en ce qu'on utilise comme produit intermédiaire la 4-(2,4-difluoro phényl)-pipérazine ou un de ses sels d'addition.

**14.** Utilisation des composés définis selon l'une quelconque des revendications 1 à 13, pour la préparation de médicaments, notamment doués de propriétés antalgiques, ou agissant sur le système nerveux central en particulier comme anxiolytique non sédatif.

**15.** Procédé de fabrication d'une compositions pharmaceutique, caractérisé en ce qu'on utilise à titre d'ingrédient actif au moins un composé selon l'une quelconque des revendications 1 à 13, que l'on mélange avec un véhicule ou excipient pharmaceutiquement acceptable.

**16.** Procédé de fabrication d'une composition pharmaceutique douée de propriétés antalgiques, caractérisé en ce qu'on utilise à titre d'ingrédient actif au moins un composé selon l'une quelconque des revendications 1 à 13, que l'on mélange avec un véhicule ou excipient pharmaceutiquement acceptable.

**17.** Procédé de fabrication d'une composition pharmaceutique agissant sur le système nerveux central, en particulier comme anxiolytique non sédatif, caractérisé en ce qu'on utilise à titre d'ingrédient actif au moins un composé selon l'une quelconque des revendica-1 à 13 que l'on mélange avec un véhicule ou excipient pharmaceutiquement acceptable.

**Revendications pour l'Etat contractant suivant: GR**

**1.** Nouveaux composés, caractérisés en ce qu'ils répondent à la formule :

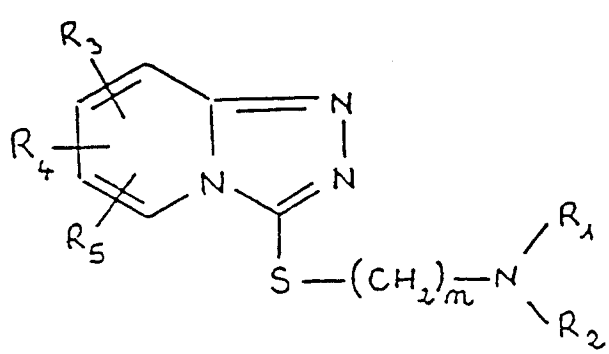

dans laquelle :

- n représente un nombre entier compris entre 1 et 8 ; optimalement 2 ou 3 ; $(CH_2)_n$-N-$R_1$ peut également former un hétérocycle à 6 atomes ;
- $R_1$ et $R_2$ peuvent représenter indépendamment l'hydrogène, un alkyle inférieur de 1 à 5 atomes de carbone, ou former ensemble avec l'atome d'azote un cycle choisi parmi la pipéridine la morpholine, la phényl tétrahydro pyridine, la pipérazine, la pipérazine N-substituée par un phényle ou un hétérocycle azoté choisi parmi pyridine et pyrimidine ; dans le cas des phényl tétrahydropyridines et des pipérazines substituées par un phényl ou un hétérocycle azoté choisi parmi pyridine et pyrimidine, le phényl ou l'hétérocycle azoté peut être substitué par des halogènes , des groupements méthoxy, hydroxy, nitro, amino, cyano, méthyle, trifluorométhyle et trichlorométhyle ;
- $R_3$, $R_4$ et $R_5$ peuvent représenter indépendamment l'hydrogène, un méthyle, un hydroxy benzyle, un halogène, un trifluorométhyle, ou deux d'entre eux peuvent former un phényle dans le cas des triazoloquinolines ;

ainsi que les sels d'addition d'acide non toxiques
ou un composé choisi parmi :

a) Dichlorhydrate hydraté de 3-[2-(4-(2,4-difluorophényl) pipérazin -1-yl) éthyl mercapto]8-(1-hydroxy éthyl)-1,2,4-triazolo-(4,3a)-pyridine ;
b) 3-[2-(4-(diphényl méthyl) pipérazin-1-yl) éthyl mercapto]-1,2,4-triazolo-(4,3a)-pyridine ; et
c) 3-[(1-benzoyl pipéridin-4-yl) mercapto]-1,2,4-triazolo-(4,3a)-pyridine.

**2.** Nouveaux composés selon la revendication 1, caractérisés en ce que $R_3 = R_4 = R_5$ = hydrogène.

**3.** Nouveaux composés selon la revendication 1, caractérisés en ce que $R_4 = R_5$ = hydrogène, $R_3$ = trifluorométhyle.

**4.** Nouveaux composés selon la revendication 1, 2 ou 3, caractérisés en ce que n = 2 ou 3.

**5.** Nouveaux composés selon l'une quelconque des revendications 1 à 4, caractérisés en ce que $NR_1R_2$ peut représenter la 4-(3-trifluoro-méthyl phényl) pipérazine.

**6.** Nouveaux composés selon l'une quelconque des revendications 1 à 4, caractérisés en ce que $NR_1R_2$ peut représenter la 4-(2,4-difluoro phényl) pipérazine.

**7.** Nouveaux composés selon l'une quelconque des revendications 1 à 4, caractérisés en ce que $NR_1R_2$ peut représenter la 4-(3-chlorophényl) pipérazine.

**8.** Nouveaux composés selon l'une quelconque des revendications 1 à 4, caractérisés en ce que $NR_1R_2$ peut représenter la 4-(2-fluorophényl) pipérazine.

**9.** Nouveau composé selon la revendication 1, caractérisé en ce qu'il répond à la formule :

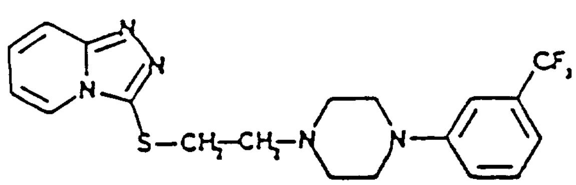

**10.** Nouveau composé selon la revendication 1, caractérisé en ce qu'il répond à la formule :

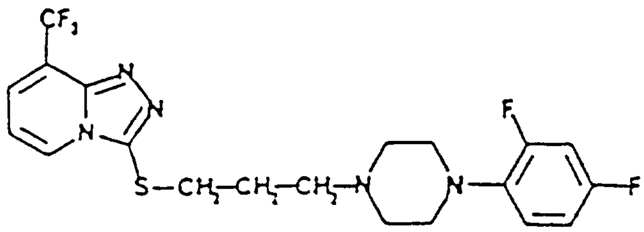

**11.** Nouveau composé selon la revendication 1, caractérisé en ce qu'il répond à la formule :

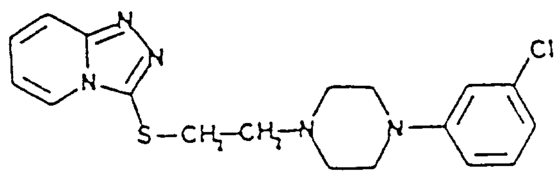

**12.** Nouveau composé selon la revendication 1, caractérisé en ce qu'il répond à la formule :

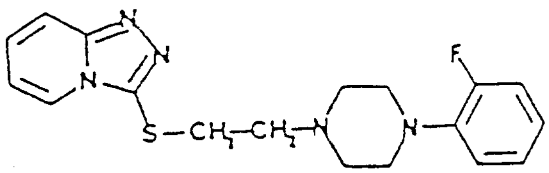

**13.** Procédé de préparation des nouveaux composés selon l'une quelconque des revendications 1 à 12, caractérisé en ce que l'on fait réagir une 3-mercapto-1,2,4,triazolo -(4,3-a) pyridine de formule II suivante :

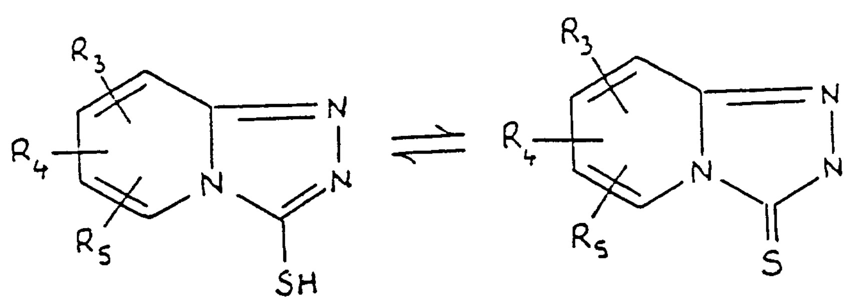

Formule (II)

dans laquelle $R_3$, $R_4$ et $R_5$ sont tels que précédemment définis, avec une halogéno-alkylamine ou une tosyloxyalkylamine ou une mésyloxyalkylamine de formule III suivante :

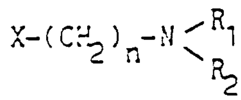

Formule (III)

dans laquelle n, $R_1$ et $R_2$ sont tels que précédemment définis, X étant un halogène ou un son groupement partant tel que tosyloxy ou mésyloxy, soit par métallation de la fonction thiol par un agent classique de métallation, soit en présence d'une base, dans un solvant organique usuel, à une température comprise entre 20 et 150°C.

**14.** Produit intermédiaire utile pour la préparation de produits de la revendication 1 qui est la 4-(2,4-difluorophényl)-pipérazine cu un de ses sels d'addition.

**15.** Procédé de fabrication d'une composition pharmaceutique, caractérisé en ce qu'on utilise à titre d'ingrédient actif au moins un composé selon l'une quelconque des revendications 1 à 12 ou 14, que l'on mélange avec un véhicule ou excipient pharmaceutiquement acceptable.

**16.** Procédé de fabrication d'une composition pharmaceutique douée de propriétés antalgiques, caractérisé en ce qu'on utilise à titre d'ingrédient actif au moins un composé selon l'une quelconque des revendications 1 à 12, ou 14, que l'on mélange avec un véhicule ou excipient pharmaceutiquement acceptable.

**17.** Procédé de fabrication d'une composition pharmaceutique agissant sur le système nerveux central, en particulier comme anxiolytique non sédatif, caractérisé en ce qu'on utilise à titre d'ingrédient actif au moins un composé selon l'une quelconque des revendications 1 à 12 ou 14, que l'on mélange avec un véhicule ou excipient pharmaceutiquement acceptable.

**Claims**
**Claims for the following Contracting States : AT, BE, CH, DE, FR, GB, IT, LI, LU, NL, SE**

**1.** Novel compounds, characterized in that they correspond to the formula:

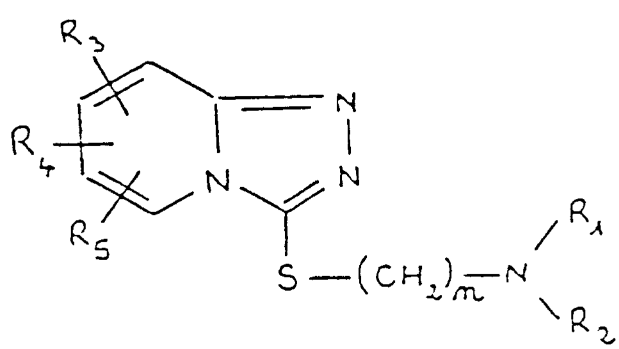

in which:

- n represents an integer between 1 and 8 and optimally 2 or 3; $(CH_2)_n$-N-$R_1$ can also form a heterocycle having 6 atoms;
- $R_1$ and $R_2$ can represent independently hydrogen, a lower alkyl having 1 to 5 carbon atoms, or can form, together with the nitrogen atom, a ring selected from the piperidine, the morpholine, the phenyltetrahydropyridine, the piperazine, the piperazine N-substituted by a phenyl or a nitrogenous heterocycle selected from pyridine and pyrimidine; in the case of the phenyltetrahydropyridines and the piperazines substituted by a phenyl or a nitrogenous heterocycle selected from pyridine and pyrimidine, the phenyl or the nitrogenous heterocycle may be substituted by halogens or methoxy, hydroxyl, nitro, amino, cyano, methyl, trifluoromethyl and

trichloromethyl groups;

- $R_3$, $R_4$ and $R_5$ can represent independently hydrogen, a methyl, a hydroxybenzyl, a halogen, a trifluoromethyl, or two of them can form a phenyl in the case of the triazoloquinolines;

as well as the non-toxic acid addition salts

or a compound selected from:

a) 3-[2-(4-(2,4-difluorophenyl)piperazin-1-yl)ethylmercapto]8-(1-hydroxyethyl)-1,2,4-triazolo-(4,3a)-pyridine hydrated dihydrochloride

b) 3-[2-(4-(diphenylmethyl)piperazin-1-yl)ethylmercapto]-1,2,4-triazolo-(4,3a)-pyridine; and

c) 3-[(1-benzoylpiperidin-4-yl)mercapto]-1,2,4-triazolo-(4,3a)-pyridine.

**2.** Novel compounds according to claim 1, characterized in that $R_3 = R_4 = R_5 =$ hydrogen.

**3.** Novel compounds according to claim 1, characterized in that $R_4 = R_5 =$ hydrogen, $R_3 =$ trifluoromethyl.

**4.** Novel compounds according to claim 1, 2 or 3, characterized in that n = 2 or 3.

**5.** Novel compounds according to any one of claims 1 to 4, characterized in that $NR_1R_2$ can represent 4-(3-trifluoromethylphenyl)piperazin.

**6.** Novel compounds according to any one of claims 1 to 4, characterized in that $NR_1R_2$ can represent 4-(2,4-difluorophenyl)piperazin.

**7.** Novel compounds according to any one of claims 1 to 4, characterized in that $NR_1R_2$ can represent 4-(3-chlorophenyl)piperazin.

**8.** Novel compounds according to any one of claims 1 to 4, characterized in that $NR_1R_2$ can represent 4-(2-fluorophenyl)piperazin.

**9.** Novel compound according to claim 1, characterized in that it corresponds to the formula:

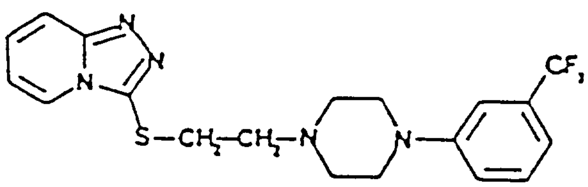

**10.** Novel compound according to claim 1, characterized in that it corresponds to the formula:

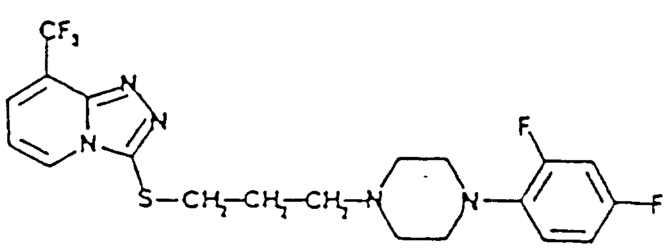

**11.** Novel compound according to claim 1, characterized in that it corresponds to the formula:

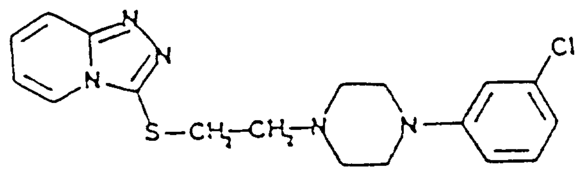

**12.** Novel compound according to claim 1, characterized in that it corresponds to the formula:

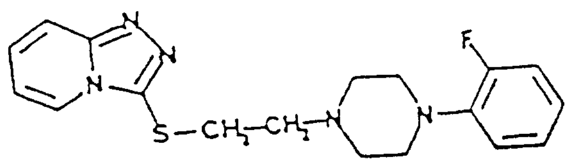

**13.** Process for the preparation of the novels compounds according to any one of claims 1 to 12, characterized in that the following 3-mercapto-1,2,4-triazolo-(4,3a) pyridine of formula II is caused to react:

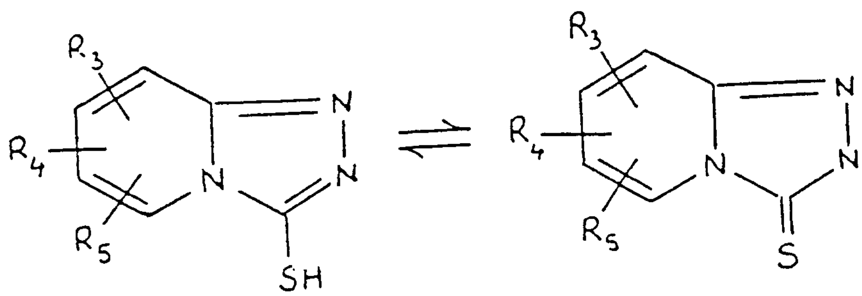

Formula (II)

in which $R_3$, $R_4$ and $R_5$ are defined as above, with a halogenoalkylamine or a tosyloxyalkylamine or a mesyloxyalkylamine of following formula III:

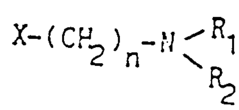

Formula (III)

in which n, $R_1$ and $R_2$ are defined as above, X being a halogen or a good leaving group such as tosyloxy or mesyloxy, either by metallating the thiol group with a conventional metallating agent, or in the presence of a base, in a customary organic solvent, at a temperature of between 20 and 150 °C.

**14.** Intermediate useful for the preparation of products of claim 1 which is 4-(2,4-difluorophenyl)piperazin or one of its addition salts.

**15.** Pharmaceutical composition, characterized in that it comprises, as the active ingredient, at least one compound according to any one of claims 1 to 12, or 14, in a pharmaceutically acceptable vehicle or excipient.

**16.** Pharmaceutical composition, characterized in that it comprises, as the active ingredient, at least one compound as prepared by the process according to claim 13, in a pharmaceutically acceptable vehicle or excipient.

**17.** Drug possessing antalgic properties, characterized in that it contains at least one compound according to any one of claims 1 to 12, or 14.

**18.** Drug acting on the central nervous system in particular as a non-sedative antianxiety medication, characterized in that it contains at least one compound according to any one of claims 1 to 12, 14.

**Claims for the following Contracting State : ES**

**1.** Process for the preparation of novel compounds corresponding to the formula:

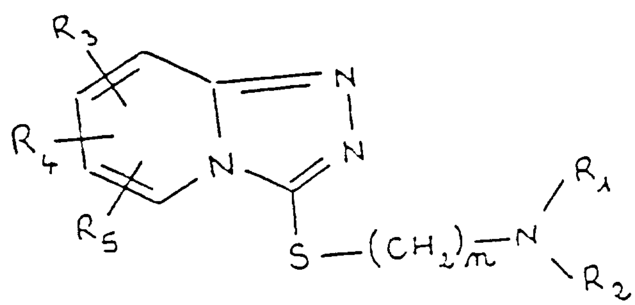

in which:
- n represents an integer between 1 and 8 and optimally 2 or 3; $(CH_2)_n$-N-$R_1$ can also form a heterocycle having 6 atoms;
- $R_1$ and $R_2$ can represent independently hydrogen, a lower alkyl having 1 to 5 carbon atoms, or can form, together with the nitrogen atom, a ring selected from the piperidine, the morpholine, the phenyltetrahydropyridine, the piperazine, the piperazine N-substituted by a phenyl or a nitrogenous heterocycle selected from pyridine and pyrimidine; in the case of the phenyltetrahydropyridines and the piperazines substituted by a phenyl or a nitrogenous heterocycle selected from pyridine and pyrimidine, the phenyl or the nitrogenous heterocycle may be substituted by halogens or methoxy, hydroxyl, nitro, amino, cyano, methyl, trifluoromethyl and trichloromethyl groups;
- $R_3$, $R_4$ and $R_5$ can represent independently hydrogen, a methyl, a hydroxybenzyl, a halogen, a trifluoromethyl, or two of them can form a phenyl in the case of the triazoloquinolines;
  as well as the non-toxic acid addition salts
  or a compound selected from:

a) 3-[2-(4-(2,4-difluorophenyl)piperazin-1-yl)ethylmercapto]8-(1-hydroxyethyl)-1,2,4-triazolo-(4,3a)-pyridine hydrated dihydrochloride
b) 3-[2-(4-(diphenylmethyl)piperazin-1-yl)ethylmercapto]-1,2,4-triazolo-(4,3a)-pyridine; and
c) 3-[(1-benzoylpiperidin-4-yl)mercapto]-1,2,4-triazolo-(4,3a)-pyridine.

characterized in that the following 3-mercapto-1,2,4-triazolo-(4,3a) pyridine of formula II :

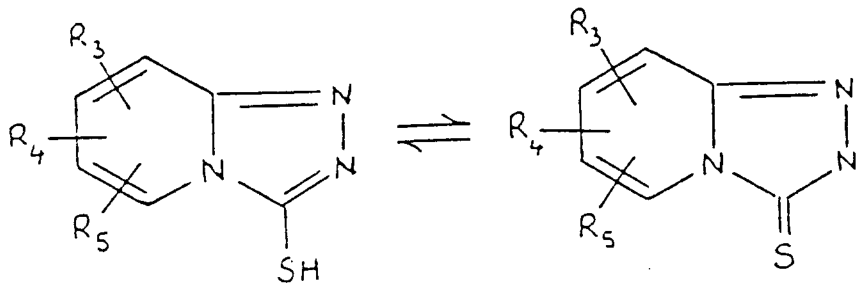

Formula (II)

is caused to react, in which $R_3$, $R_4$ and $R_5$ are defined as above, with a halogenoalkylamine or a tosyloxyalkylamine or a mesyloxyalkylamine of following formula III:

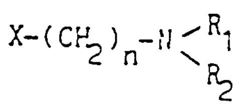

Formula (III)

in which n, $R_1$ and $R_2$ are defined as above, X being a halogen or a good leaving group such as tosyloxy or mesyloxy, either by metallating the thiol group with a conventional metallating agent, or in the presence of a base, in a customary organic solvent, at a temperature of between 20 and 150 ° C.

2. Process according to claim 1, characterized in that the compound whereby $R_3 = R_4 = R_5$ = hydrogen is prepared.

3. Process according to claim 1, characterized in that the compound whereby $R_4 = R_5$ = hydrogen, $R_3$ = trifluoromethyl is prepared.

4. Process according to claim 1, 2 or 3, characterized in that the compounds whereby n = 2 or 3 are prepared.

5. Process according to any one of claims 1 to 4, characterized in that the compounds whereby $NR_1R_2$ can represent 4-(3-trifluoromethyl- phenyl)piperazin are prepared.

6. Process according to any one of claims 1 to 4, characterized in that the compounds whereby $NR_1R_2$ can represent 4-(2,4-difluorophenyl)piperazin are prepared.

7. Process according to any one of claims 1 to 4, characterized in that the compounds whereby $NR_1R_2$ can represent 4-(3-chlorophenyl)piperazin are prepared.

8. Process according to any one of claims 1 to 4, characterized in that the compounds whereby $NR_1R_2$ can represent 4-(2-fluorophenyl)piperazin are prepared.

9. Process according to claim 1, characterized in that the compound corresponding to the formula:

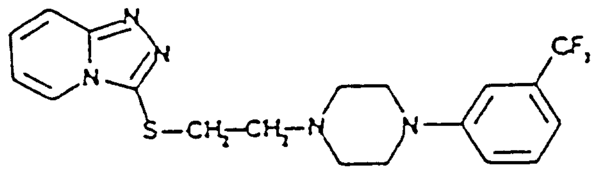

is prepared.

10. Process according to claim 1, characterized in that the compound corresponding to the formula:

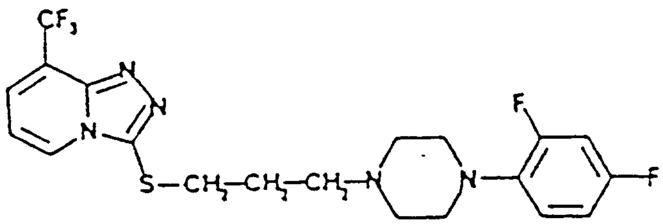

is prepared.

11. Process according to claim 1, characterized in that the compound corresponding to the formula:

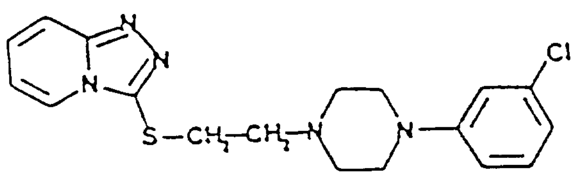

is prepared.

12. Process according to claim 1, characterized in that the compound corresponding to the formula:

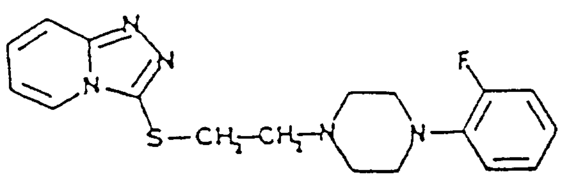

is prepared.

13. Process for the preparation of the compounds according to any one of claims 1 to 12, characterized in that 4-(2,4-difluorophenyl)piperazin or one of its addition salts is used as an intermediate.

14. Use of the compounds defined according to any one of claims 1 to 13, for the preparation of drugs, in particular possessing antalgic properties, or acting on the central nervous system in particular as a non-sedative antianxiety medication.

**15.** Process of manufacture of a pharmaceutical composition, characterized in that at least one compound according to any one of claims 1 to 13, is used as the active ingredient in admixture with a pharmaceutically acceptable vehicle or excipient.

**16.** Process of manufacture of a pharmaceutical composition possessing antalgic properties, characterized in that at least one compound according to any one of claims 1 to 13, is used as the active ingredient in admixture with a pharmaceutically acceptable vehicle or excipient.

**17.** Process of manufacture of a pharmaceutical composition acting on the central nervous system, in particular as a non-sedative antianxiety medication, characterized in that at least one compound according to any one of claims 1 to 13, is used as the active ingredient in admixture with a pharmaceutically acceptable vehicle or excipient.

**Claims for the following Contracting State : GR**

**1.** Novel compounds, characterized in that they correspond to the formula:

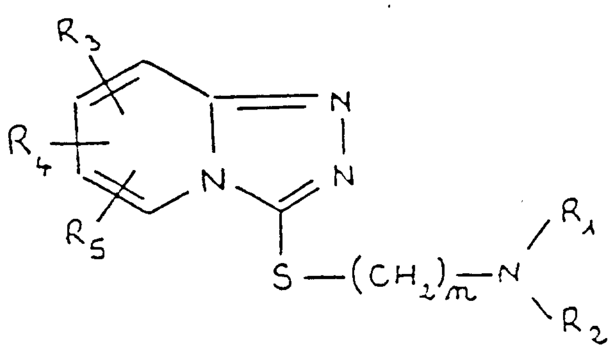

in which:

- n represents an integer between 1 and 8 and optimally 2 or 3; $(CH_2)_n$-N-$R_1$ can also form a heterocycle having 6 atoms;
- $R_1$ and $R_2$ can represent independently hydrogen, a lower alkyl having 1 to 5 carbon atoms, or can form, together with the nitrogen atom, a ring selected from the piperidine, the morpholine, the phenyltetrahydropyridine, the piperazine, the piperazine N-substituted by a phenyl or a nitrogenous heterocycle selected from pyridine and pyrimidine; in the case of the phenyltetrahydropyridines and the piperazines substituted by a phenyl or a nitrogenous heterocycle selected from pyridine and pyrimidine, the phenyl or the nitrogenous heterocycle may be substituted by halogens or methoxy, hydroxyl, nitro, amino, cyano, methyl, trifluoromethyl and trichloromethyl groups;
- $R_3$, $R_4$ and $R_5$ can represent independently hydrogen, a methyl, a hydroxybenzyl, a halogen, a trifluoromethyl, or two of them can form a phenyl in the case of the triazoloquinolines;
  as well as the non-toxic acid addition salts
  or a compound selected from:

a) 3-[2-(4-(2,4-difluorophenyl)piperazin-1-yl)ethylmercapto]8-(1-hydroxyethyl)-1,2,4-triazolo-(4,3a)-pyridine hydrated dihydrochloride

b) 3-[2-(4-(diphenylmethyl)piperazin-1-yl)ethylmercapto]-1,2,4-triazolo-(4,3a)-pyridine; and

c) 3-[(1-benzoylpiperidin-4-yl)mercapto]-1,2,4-triazolo-(4,3a)-pyridine.

**2.** Novel compounds according to claim 1, characterized in that $R_3 = R_4 = R_5$ = hydrogen.

**3.** Novel compounds according to claim 1, characterized in that $R_4 = R_5$ = hydrogen, $R_3$ = trifluoromethyl.

**4.** Novel compounds according to claim 1, 2 or 3, characterized in that n = 2 or 3.

5. Novel compounds according to any one of claims 1 to 4, characterized in that $NR_1R_2$ can represent 4-(3-trifluoromethyl-phenyl)piperazin.

6. Novel compounds according to any one of claims 1 to 4, characterized in that $NR_1R_2$ can represent 4-(2,4-difluorophenyl)piperazin.

7. Novel compounds according to any one of claims 1 to 4, characterized in that $NR_1R_2$ can represent 4-(3-chlorophenyl)piperazin.

8. Novel compounds according to any one of claims 1 to 4, characterized in that $NR_1R_2$ can represent 4-(2-fluorophenyl)piperazin.

9. Novel compound according to claim 1, characterized in that it corresponds to the formula:

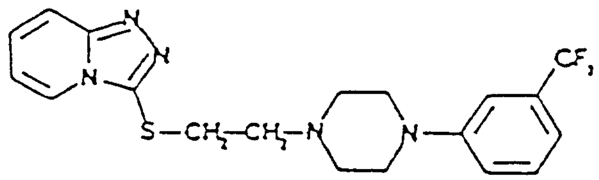

10. Novel compound according to claim 1, characterized in that it corresponds to the formula:

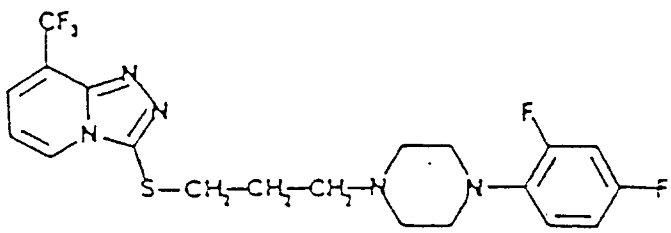

11. Novel compound according to claim 1, characterized in that it corresponds to the formula:

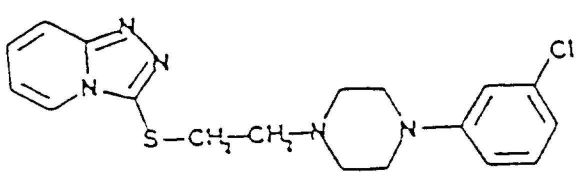

12. Novel compound according to claim 1, characterized in that it corresponds to the formula:

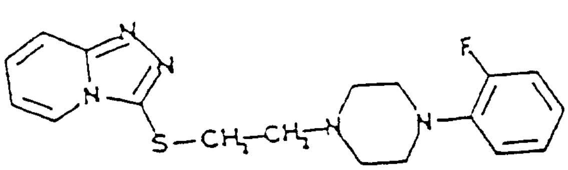

13. Process for the preparation of the novels compounds according to any one of claims 1 to 12, characterized in that the following 3-mercapto-1,2,4-triazolo-(4,3a) pyridine of formula II is caused to react:

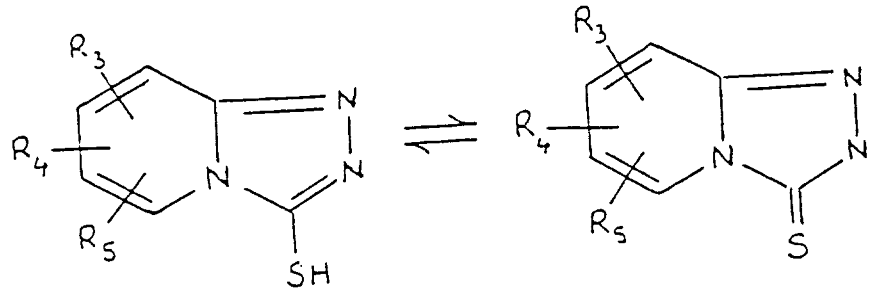

Formula (II)

in which $R_3$, $R_4$ and $R_5$ are defined as above, with a halogenoalkylamine or a tosyloxyalkylamine or a mesyloxyalkylamine of following formula III:

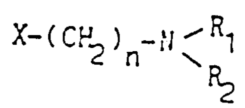

Formula (III)

in which n, $R_1$ and $R_2$ are defined as above, X being a halogen or a good leaving group such as tosyloxy or mesyloxy, either by metallating the thiol group with a conventional metallating agent, or in the presence of a base, in a customary organic solvent, at a temperature of between 20 and 150 °C.

14. Intermediate useful for the preparation of products of claim 1 which is 4-(2,4-difluorophenyl)piperazin or one of its addition salts.

15. Process of manufacture of a pharmaceutical composition, characterized in that at least one compound according to any one of claims 1 to 12, or 14, is used as the active ingredient in admixture with a pharmaceutically acceptable vehicle or excipient.

16. Process of manufacture of a pharmaceutical composition possessing antalgic properties, characterized in that at least one compound according to any one of claims 1 to 12, or 14, is used as the active ingredient in admixture with a pharmaceutically acceptable vehicle or excipient.

17. Process of manufacture of a pharmaceutical composition acting on the central nervous system, in particular as a non-sedative antianxiety medication, characterized in that at least one compound according to any one of claims 1 to 12, or 14, is used as the active ingredient in admixture with a pharmaceutically acceptable vehicle or excipient.

## Patentansprüche

**Patentansprüche für folgende Vertragsstaaten : AT, BE, CH, DE, FR, GB, IT, LI, LU, NL, SE**

1. Neue Verbindungen, dadurch gekennzeichnet, daß sie der Formel

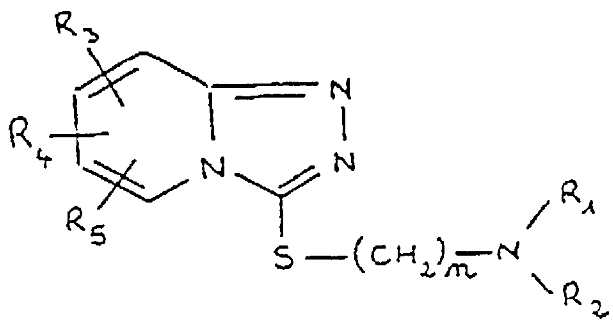

entsprechen, worin:

- n eine ganze Zahl zwischen 1 und 8, am besten 2 oder 3, ist; $(CH_2)_n$-N-$R_1$ auch einen Heterozyklus mit 6 Atomen bilden kann;
- $R_1$ und $R_2$ unabhängig voneinander Wasserstoff oder ein nied.Alkyl mit 1 bis 5 Kohlenstoffatomen darstellen oder zusammen mit dem Stickstoffatom einen Ring, ausgewählt aus Piperidin, Morpholin, Phenyltetrahydropyridin, Piperazin, durch ein Phenyl oder einen Stickstoffheterozyklus, ausgewählt aus Pyridin und Pyrimidin, N-substituiertes Piperazin bilden können; im Fall von Phenyltetrahydropyridinen und Piperazinen, die durch ein Phenyl oder einen Stickstoffheterozyklus, ausgewählt aus Pyridin und Pyrimidin, substituiert sind, kann das Phenyl oder der Stickstoffheterozyklus durch Halogene, Methoxy-, Hydroxy-, Nitro-, Amino-, Cyano-, Methyl-, Trifluormethyl- und Trichlormethylgruppen substituiert sein;
- $R_3$, $R_4$ und $R_5$ unabhängig voneinander Wasserstoff, Methyl, Hydroxybenzyl, Halogen oder Trifluormethyl darstellen können, oder im Fall von Triazolochinolinen zwei von ihnen ein Phenyl bilden können;

sowie die Additionssalze von nicht toxischen Säuren

oder eine Verbindung, ausgewählt aus:

a) 3-[2-(4-(2,4-Difluorphenyl)-piperazin-1-yl)-ethylmercapto]-8-(1-hydroxyethyl)-1,2,4-triazolo-(4,3a)-pyridin-dihydrochloridhydrat;

b) 3-[2-(4-(Diphenylmethyl)-piperazin-1-yl)-ethylmercapto]-1,2,4-triazolo-(4,3a)-pyridin; und

c) 3-[(1-Benzoylpiperidin-4-yl)-mercapto]-1,2,4-triazolo-(4,3a)-pyridin.

2. Neue Verbindungen nach Anspruch 1, dadurch gekennzeichnet, daß $R_3 = R_4 = R_5$ = Wasserstoff.

3. Neue Verbindungen nach Anspruch 1, dadurch gekennzeichnet, daß $R_4 = R_5$ = Wasserstoff, $R_3$ = Trifluormethyl.

4. Neue Verbindungen nach Anspruch 1, 2 oder 3, dadurch gekennzeichnet, daß n = 2 oder 3.

5. Neue Verbindungen nach einem der Ansprüche 1 bis 4, dadurch gekennzeichnet, daß $NR_1R_2$ 4-(3-Trifluormethylphenyl)-piperazin darstellen kann.

6. Neue Verbindungen nach einem der Ansprüche 1 bis 4, dadurch gekennzeichnet, daß $NR_1R_2$ 4-(2,4-Difluorphenyl)-piperazin darstellen kann.

7. Neue Verbindungen nach einem der Ansprüche 1 bis 4, dadurch gekennzeichnet, daß $NR_1R_2$ 4-(3-Chlorphenyl)-piperazin darstellen kann.

8. Neue Verbindungen nach einem der Ansprüche 1 bis 4, dadurch gekennzeichnet, daß $NR_1R_2$ 4-(2-Fluorphenyl)-piperazin darstellen kann.

9. Neue Verbindung nach Anspruch 1, dadurch gekennzeichnet, daß sie der Formel

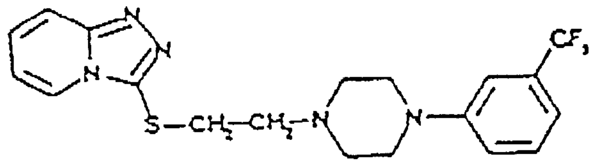

entspricht.

10. Neue Verbindung nach Anspruch 1, dadurch gekennzeichnet, daß sie der Formel

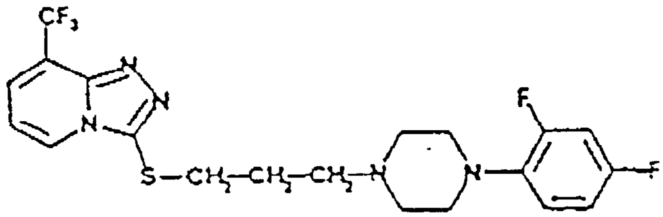

entspricht.

11. Neue Verbindung noch Anspruch 1, dadurch gekennzeichnet, daß sie der Formel

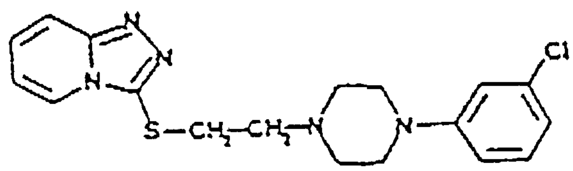

entspricht.

12. Neue Verbindung nach Anspruch 1, dadurch gekennzeichnet, daß sie der Formel

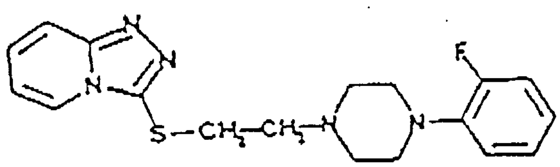

entspricht.

13. Verfahren zur Herstellung der neuen Verbindungen nach einem der Ansprüche 1 bis 12, dadurch gekennzeichnet, daß ein 3-Mercapto-1,2,4-triazolo-(4,3a)-pyridin der nachstehenden Formel II:

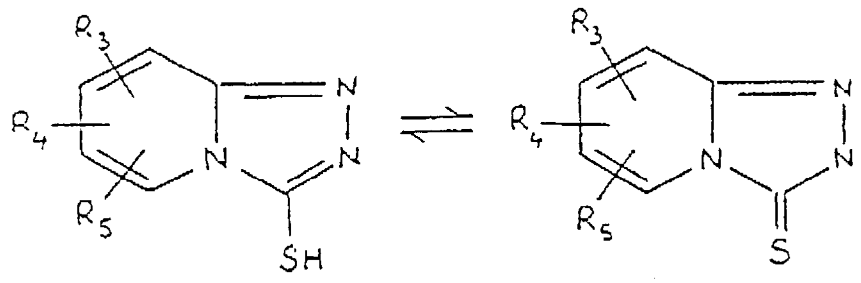

Formel (II)

worin $R_3$, $R_4$ und $R_5$ wie zuvor definiert sind, mit einem Halogenalkylamin oder einem Tosyloxyalkylamin oder einem Mesyloxyalkylamin der nachstehenden Formel III:

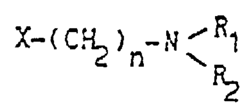

Formel (III)

worin n, $R_1$ und $R_2$ wie zuvor definiert sind, wobei X ein Halogen oder eine gute Abgangsgruppe, wie Tosyloxy oder Mesyloxy, ist, entweder durch Metallierung der Thiolfunktion mit einem klassischen Metallierungsmittel oder in Gegenwart einer Base in einem üblichen organischen Lösungsmittel bei einer Temperatur zwischen 20 und 150 °C reagieren gelassen wird.

**14.** Zwischenprodukt, das zur Herstellung von Produkten des Anspruchs 1 geeignet ist, nämlich 4-(2,4-Difluorphenyl)-piperazin oder eines seiner Additionssalze.

**15.** Pharmazeutische Zusammensetzung, dadurch gekennzeichnet, daß sie als aktives Ingrediens mindestens eine Verbindung nach einem der Ansprüche 1 bis 12 oder 14 in einem pharmazeutisch akzeptablen Vehikel oder Exzipienten enthält.

**16.** Pharmazeutische Zusammensetzung, dadurch gekennzeichnet, daß sie als aktives Ingrediens mindestens eine gemäß dem Verfahren nach Anspruch 13 hergestellte Verbindung in einem pharmazeutisch akzeptablen Vehikel oder Exzipienten enthält.

**17.** Medikament mit analgetischen Eigenschaften, dadurch gekennzeichnet, daß es mindestens eine Verbindung nach einem der Ansprüche 1 bis 12 oder 14 enthält.

**18.** Medikament mit Wirkung auf das Zentralnervensystem, insbesondere als nicht sedatives Anxiolytikum, dadurch gekennzeichnet, daß es mindestens eine Verbindung nach einem der Ansprüche 1 bis 12 oder 14 enthält.

**Patentansprüche für folgenden Vertragsstaat : ES**

**1.** Verfahren zur Herstellung neuer Verbindungen der Formel

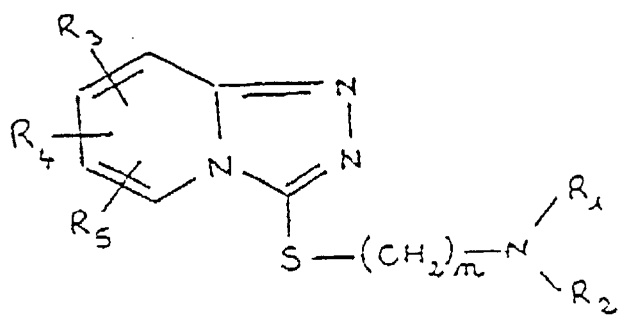

worin:

- n eine ganze Zahl zwischen 1 und 8, am besten 2 oder 3, ist; $(CH_2)_n$-N-$R_1$ auch einen Heterozyklus mit 6 Atomen bilden kann;
- $R_1$ und $R_2$ unabhängig voneinander Wasserstoff oder ein nied.Alkyl mit 1 bis 5 Kohlenstoffatomen darstellen oder zusammen mit den Stickstoffatom einen Ring, ausgewählt aus Piperidin, Morpholin, Phenyltetrahydropyridin, Piperazin, durch ein Phenyl oder einen Stickstoffheterozyklus, ausgewählt aus Pyridin und Pyrimidin, N-substituiertes Piperazin bilden können; im Fall von Phenyltetrahydropyridinen und Piperazinen, die durch ein Phenyl oder einen Stickstoffheterozyklus, ausgewählt aus Pyridin und Pyrimidin, substituiert sind, kann das Phenyl oder der Stickstoffheterozyklus durch Halogene, Methoxy-, Hydroxy-, Nitro-, Amino-, Cyano-, Methyl-, Trifluormethyl- und Trichlormethylgruppen substituiert sein;
- $R_3$, $R_4$ und $R_5$ unabhängig voneinander Wasserstoff, Methyl, Hydroxybenzyl, Halogen oder Trifluormethyl darstellen können, oder im Fall von Triazolochinolinen zwei von ihnen ein Phenyl bilden können;

sowie die Additionssalze von nicht toxischen Säuren
oder eine Verbindung, ausgewählt aus:

a) 3-[2-(4-(2,4-Difluorphenyl)-piperazin-1-yl)-ethylmercapto]-8-(1-hydroxyethyl)-1,2,4-triazolo-(4,3a)-pyridin-dihydrochloridhydrat;
b) 3-[2-(4-(Diphenylmethyl)-piperazin-1-yl)-ethylmercapto]-1,2,4-triazolo-(4,3a)-pyridin; und
c) 3-[(1-Benzoylpiperidin-4-yl)-mercapto]-1,2,4-triazolo-(4,3a)-pyridin,

dadurch gekennzeichnet, daß ein 3-Mercapto-1,2,4-triazolo-(4,3a)-pyridin der nachstehenden Formel II:

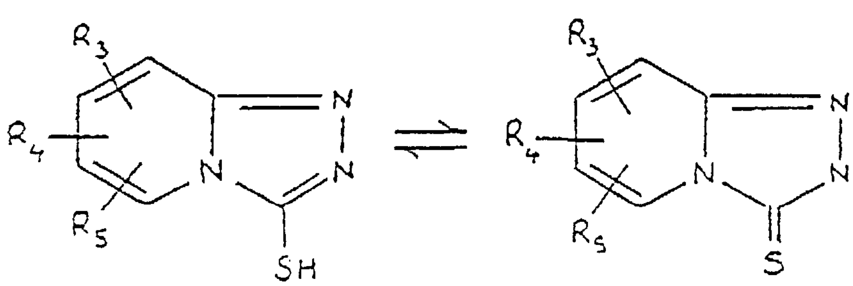

Formel (II)

worin $R_3$, $R_4$ und $R_5$ wie zuvor definiert sind, mit einem Halogenalkylamin oder einem Tosyloxyalkylamin oder einem Mesyloxyalkylamin dar nachstehenden Formel III:

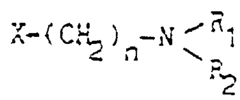

Formel (III)

worin n, $R_1$ und $R_2$ wie zuvor definiert sind, wobei X ein Halogen oder eine gute Abgangsgruppe, wie Tosyloxy oder Mesyloxy, ist, entweder durch Metallierung der Thiolfunktion mit einem klassischen Metallierungsmittel oder in Gegenwart einer Base in einem üblichen organischen Lösungsmittel bei einer Temperatur zwischen 20 und 150 °C reagieren gelassen wird.

**2.** Verfahren nach Anspruch 1, dadurch gekennzeichnet, daß man die Verbindung herstellt, in der $R_3$ = $R_4$ = $R_5$ = Wasserstoff.

**3.** Verfahren nach Anspruch 1, dadurch gekennzeichnet, daß man die Verbindung herstellt, in der $R_4$ = $R_5$ = Wasserstoff, $R_3$ = Trifluormethyl.

**4.** Verfahren nach Anspruch 1, 2 oder 3, dadurch gekennzeichnet, daß man die Verbindungen herstellt, in denen n = 2 oder 3.

**5.** Verfahren nach einem der Ansprüche 1 bis 4, dadurch gekennzeichnet, daß man die Verbindungen herstellt, in denen $NR_1R_2$ 4-(3-Trifluormethylphenyl)-piperazin darstellen kann.

**6.** Verfahren nach einem der Ansprüche 1 bis 4, dadurch gekennzeichnet, daß man die Verbindungen herstellt, in denen $NR_1R_2$ 4-(2,4-Difluorphenyl)-piperazin darstellen kann.

**7.** Verfahren nach einem der Ansprüche 1 bis 4, dadurch gekennzeichnet, daß man die Verbindungen herstellt, in denen $NR_1R_2$ 4-(3-Chlorphenyl)-piperazin darstellen kann.

**8.** Verfahren nach einem der Ansprüche 1 bis 4, dadurch gekennzeichnet, daß man die Verbindungen herstellen kann, in denen $NR_1R_2$ 4-(2-Fluorphenyl)-piperazin darstellen kann.

**9.** Verfahren nach Anspruch 1, dadurch gekennzeichnet, daß man die Verbindung der Formel

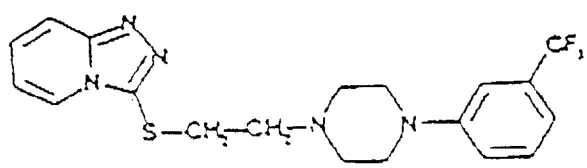

herstellt.

**10.** Verfahren nach Anspruch 1, dadurch gekennzeichnet, daß man die Verbindung der Formel

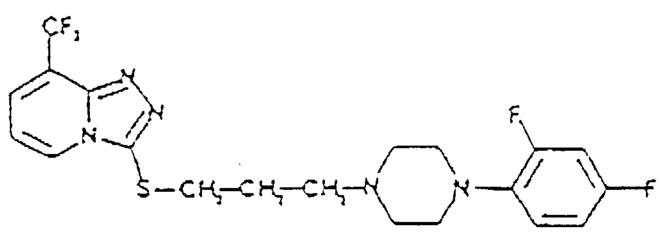

herstellt.

**11.** Verfahren nach Anspruch 1, dadurch gekennzeichnet, daß man die Verbindung der Formel

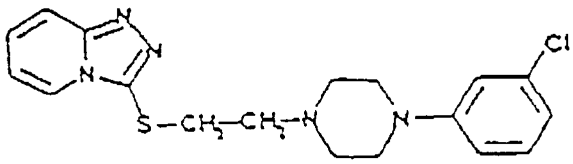

herstellt.

**12.** Verfahren nach Anspruch 1, dadurch gekennzeichnet, daß man die Verbindung der Formel

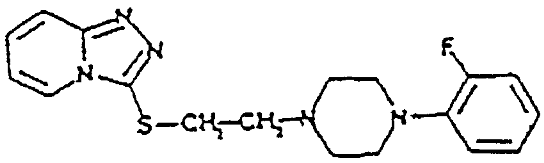

herstellt.

**13.** Verfahren zur Herstellung der Verbindungen nach einem der Ansprüche 1 bis 12, dadurch gekennzeichnet, daß man als Zwischenprodukt 4-(2,4-Difluorphenyl)-piperazin oder eines seiner Additionssalze verwendet.

**14.** Verwendung der Verbindungen nach einem der Ansprüche 1 bis 13 zur Herstellung von Medikamenten, die insbesondere mit analgetischen Eigenschaften ausgestattet sind oder auf das Zentralnervensystem, insbesondere als nicht sedatives Anxiolytikum, wirken.

**15.** Verfahren zur Herstellung einer pharmazeutischen Zusammensetzung, dadurch gekennzeichnet, daß man als aktives Ingrediens mindestens eine Verbindung nach einem der Ansprüche 1 bis 13 verwendet, die man mit einem pharmazeutisch akzeptablen Vehikel oder Exzipienten mischt.

**16.** Verfahren zur Herstellung einer pharmazeutischen Zusammensetzung mit analgetischen Eigenschaften, dadurch gekennzeichnet, daß man als aktives Ingrediens mindestens eine Verbindung nach einem der Ansprüche 1 bis 13 verwendet, die man mit einem pharmazeutisch akzeptablen Vehikel oder Exzipienten mischt.

**17.** Verfahren zur Herstellung einer pharmazeutischen Zusammensetzung mit Wirkung auf das Zentralnervensystem, insbesondere als nicht sedatives Anxiolytikum, dadurch gekennzeichnet, daß man als aktives Ingrediens mindestens eine Verbindung nach einem der Ansprüche 1 bis 13 verwendet, die man mit einem pharmazeutisch akzeptablen Vehikel oder Exzipienten mischt.

**Patentansprüche für folgenden Vertragsstaat : GR**

1. Neue Verbindungen, dadurch gekennzeichnet, daß sie der Formel

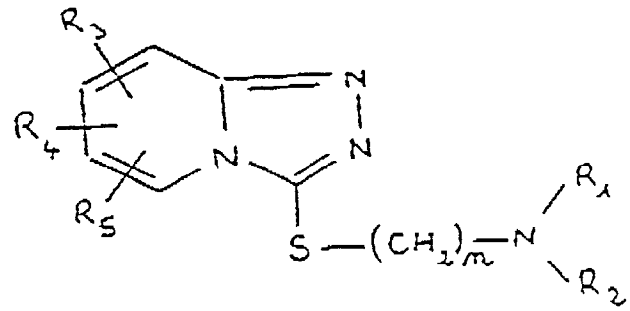

entsprechen, worin:

- n eine ganze Zahl zwischen 1 und 8, am besten 2 oder 3, ist; $(CH_2)_n$-N-$R_1$ auch einen Heterozyklus mit 6 Atomen bilden kann;
- $R_1$ und $R_2$ unabhängig voneinander Wasserstoff oder ein nied.Alkyl mit 1 bis 5 Kohlenstoffatomen darstellen oder zusammen mit dem Stickstoffatom einen Ring, ausgewählt aus Piperidin, Morpholin, Phenyltetrahydropyridin, Piperazin, durch ein Phenyl oder einen Stickstoffheterozyklus, ausgewählt aus Pyridin und Pyrimidin, N-substituiertes Piperazin bilden können; im Fall von Phenyltetrahydropyridinen und Piperazinen, die durch ein Phenyl oder einen Stickstoffheterozyklus, ausgewählt aus Pyridin und Pyrimidin, substituiert sind, kann das Phenyl oder der Stickstoffheterozyklus durch Halogene, Methoxy-, Hydroxy-, Nitro-, Amino-, Cyano-, Methyl-, Trifluormethyl- und Trichlormethylgruppen substituiert sein;
- $R_3$, $R_4$ und $R_5$ unabhängig voneinander Wasserstoff, Methyl, Hydroxybenzyl, Halogen oder Trifluormethyl darstellen können, oder im Fall von Triazolochinolinen zwei von ihnen ein Phenyl bilden können;

sowie die Additionssalze von nicht toxischen Säuren
oder eine Verbindung, ausgewählt aus;

a) 3-[2-(4-(2,4-Difluorphenyl)-piperazin-1-yl)-ethylmercapto]-8-(1-hydroxyethyl)-1,2,4-triazolo-(4,3a)-pyridin-dihydrochloridhydrat;
b) 3-[2-(4-(Diphenylmethyl)-piperazin-1-yl)-ethylmercapto]-1,2,4-triazolo-(4,3a)-pyridin; und
c) 3-[(1-Benzoylpiperidin-4-yl)-mercapto]-1,2,4-triazolo-(4,3a)-pyridin.

2. Neue Verbindungen nach Anspruch 1, dadurch gekennzeichnet, daß $R_3 = R_4 = R_5$ = Wasserstoff.

3. Neue Verbindungen nach Anspruch 1, dadurch gekennzeichnet, daß $R_4 = R_5$ = Wasserstoff, $R_3$ = Trifluormethyl.

4. Neue Verbindungen nach Anspruch 1, 2 oder 3, dadurch gekennzeichnet, daß n = 2 oder 3.

5. Neue Verbindungen nach einem der Ansprüche 1 bis 4, dadurch gekennzeichnet, daß $NR_1R_2$ 4-(3-Trifluormethylphenyl)-piperazin darstellen kann.

6. Neue Verbindungen nach einem der Ansprüche 1 bis 4, dadurch gekennzeichnet, daß $NR_1R_2$ 4-(2,4-Difluorphenyl)-piperazin darstellen kann.

7. Neue Verbindungen nach einem der Ansprüche 1 bis 4, dadurch gekennzeichnet, daß $NR_1R_2$ 4-(3-Chlorphenyl)-piperazin darstellen kann.

8. Neue Verbindungen nach einem der Ansprüche 1 bis 4, dadurch gekennzeichnet, daß $NR_1R_2$ 4-(2-Fluorphenyl)-piperazin darstellen kann.

**9.** Neue Verbindung nach Anspruch 1, dadurch gekennzeichnet, daß sie der Formel

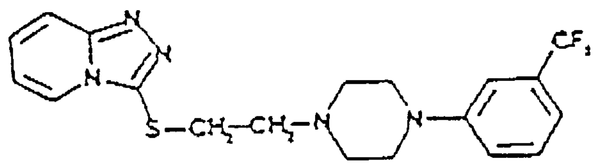

entspricht.

**10.** Neue Verbindung nach Anspruch 1, dadurch gekennzeichnet, daß sie der Formel

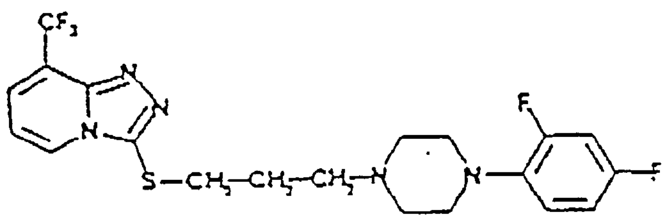

entspricht.

**11.** Neue Verbindung nach Anspruch 1, dadurch gekennzeichnet, daß sie der Formel

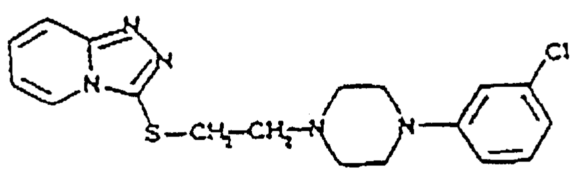

entspricht.

**12.** Neue Verbindung nach Anspruch 1, dadurch gekennzeichnet, daß sie der Formel

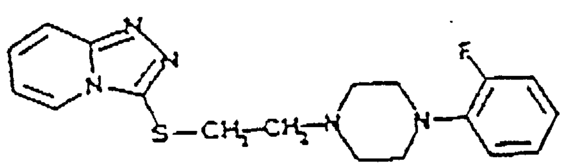

entspricht.

**13.** Verfahren zur Herstellung der neuen Verbindungen nach einem der Ansprüche 1 bis 12, dadurch gekennzeichnet daß ein 3-Mercapto-1,2,4-triazolo-(4,3a)-pyridin der nachstehenden Formel II:

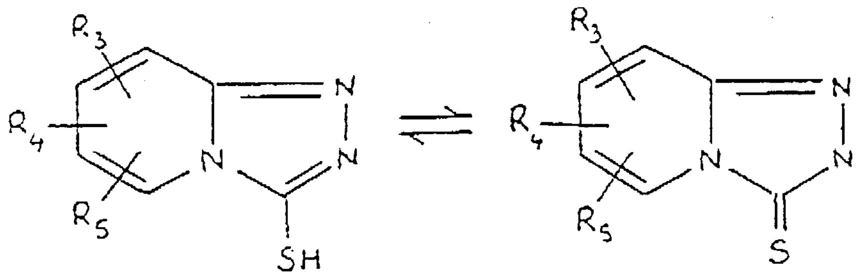

Formel (II)

worin $R_3$, $R_4$ und $R_5$ wie zuvor definiert sind, mit einem Halogenalkylamin oder einem Tosyloxyalkylamin oder einem Mesyloxyalkylamin der nachstehenden Formel III:

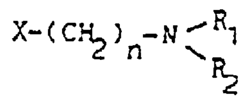

Formel (III)

worin n, $R_1$ und $R_2$ wie zuvor definiert sind, wobei X ein Halogen oder eine gute Abgangsgruppe, wie Tosyloxy oder Mesyloxy, ist, entweder durch Metallierung der Thiolfunktion mit einem klassischen Metallierungsmittel oder in Gegenwart einer Base in einem üblichen organischen Lösungsmittel bei einer Temperatur zwischen 20 und 150 °C reagieren gelassen wird.

**14.** Zwischenprodukt, das zur Herstellung von Produkten des Anspruchs 1 geeignet ist, nämlich 4-(2,4-Difluorphenyl)-piperazin oder eines seiner Additionssalze.

**15.** Verfahren zur Herstellung einer pharmazeutischen Zusammensetzung, dadurch gekennzeichnet, daß man als aktives Ingrediens mindestens eine Verbindung nach einem der Ansprüche 1 bis 12 oder 14 verwendet, die man mit einem pharmazeutisch akzeptablen Vehikel oder Enzipienten mischt.

**16.** Verfahren zur Herstellung einer pharmazeutischen Zusammensetzung mit analgetischen Eigenschaften, dadurch gekennzeichnet, daß man als aktives Ingrediens mindestens eine Verbindung nach einem der Ansprüche 1 bis 12 oder 14 verwendet, die man mit einem pharmazeutisch akzeptablen Vehikel oder Exzipienten mischt.

**17.** Verfahren zur Herstellung einer pharmazeutischen Zusammensetzung mit Wirkung auf das Zentralnervensystem, insbesondere als nicht sedatives Anxiolytikum, dadurch gekennzeichnet, daß man als aktives Ingrediens mindestens eine Verbindung nach einem der Ansprüche 1 bis 12 oder 14 verwendet, die man mit einem pharmazeutisch akzeptablen Vehikel oder Exzipienten mischt.